# EUROPÄISCHE PATENTSCHRIFT

(11) **EP 3 441 765 B1**
(45) Veröffentlichungstag und Bekanntmachung des Hinweises auf die Patenterteilung: **20.10.2021**
(21) Anmeldenummer: 18187647.5
(22) Anmeldetag: 07.08.2018
(51) Int. Cl.: G01N 33/50

(54) **VERFAHREN ZUM NACHWEIS EINER BASOPHILENAKTIVIERUNG**
METHOD FOR DETECTING BASOPHIL ACTIVATION
PROCÉDÉ DE DÉTECTION D'UNE ACTIVATION DE CELLULES BASOPHILES

(30) Priorität: 08.08.2017 EP 17001352; 05.12.2017 EP 17001979
(43) Veröffentlichungstag der Anmeldung: 13.02.2019
(73) Patentinhaber: Euroimmun Medizinische Labordiagnostika AG, 23560 Lübeck (DE)
(72) Erfinder: Gruhne, Julia, 22769 Hamburg (DE); Seismann, Henning, 23863 Nienwohld (DE); Weimann, Alf, 23560 Lübeck (DE)

(56) Entgegenhaltungen:
- EP-A1- 2 037 269
- WO-A2-2012/044756
- US-A- 4 435 509
- Anonymus: "Basotest Reagent kit for the quantitative determination of the degranulation of basophilic granulocytes in heparinized human whole blood", online , 4. August 2016 (2016-08-04), XP002774283, Gefunden im Internet: URL:http://www.bdbiosciences.com/ds/pm/tds /340547.pdf [gefunden am 2017-09-29]
- SAARNE T ET AL: "Rational design of hypoallergens applied to the major cat allergen Fel d 1", CLINICAL & EXPERIMENTAL ALLERGY : JOURNAL OF THE BRITISH SOCIETY FOR ALLERGY AND CLINICAL IMMUNOLOGY, WILEY INTERSCIENCE, UK, Bd. 35, Nr. 5, 1. Mai 2005 (2005-05-01), Seiten 657-663, XP002397061, ISSN: 0954-7894, DOI: 10.1111/J.1365-2222.2005.02234.X

## Beschreibung

Hiermit wird ein Verfahren zur Diagnose einer Allergie, umfassend die Schritte Kontaktieren von Basophilen aus einer Vollblutprobe eines Patienten mit einem Allergen in einer wässrigen Lösung in Anwesenheit des Vollblutes mit einem Volumenanteil des Vollblutes an der wässrigen Lösung von wenigstens 20 Volumenprozent unter Bedingungen, die eine Aktivierung der Basophilen durch das Allergen zulassen, Anreichern der Basophilen aus a) und Nachweisen aktivierter Basophiler, wobei das Nachweisen dadurch erreicht wird, dass die ein für aktivierte Basophile charakteristischer Marker mittels Chemilumineszenz detektiert wird; und ein Kit umfassend ein Gefäß zur Kontaktierung der Basophilen mit dem Allergen, wobei es sich bei dem Gefäß bevorzugt um eine Mikrotiterplatte handelt, optional ein Allergen, ein Mittel zur Detektion eines für aktivierte Basophile charakteristischen Markers mittels Chemilumineszenz, wobei es sich bevorzugt um einen mit einer chemilumineszenzfähigen Markierung versehenen Liganden gegen den Marker handelt und der Ligand noch bevorzugter ein monoklonaler Antikörper ist, optional ein Mittel zum Stoppen der Aktivierung der Basophilen, einen optional immobilisierbaren Liganden, der an ein unabhängig von ihrem Aktivierungszustand an der Zelloberfläche von Basophilen lokalisiertes Polypeptid bindet, wobei es sich bei dem Liganden bevorzugt um einen monoklonalen Antikörper gegen das Polypeptid handelt und optional ein Mittel zum Lysieren und/oder Entfernen von Erythrozyten, offenbart.

Die vorliegende Erfindung betrifft ein Verfahren gemäß Anspruch 1, ein Kit gemäß Anspruch 16 und eine Verwendung gemäß Anspruch 17.

Zur Diagnose von Allergien des Soforttyps, auch als Typ I-Allergien bezeichnet, die auf einer IgE-vermittelte Immunantwort basieren, werden nach einer ausführlichen Anamnese vorrangig Hauttests wie Skin-Prick-Tests oder Provokationstests durchgeführt, die für die Allergiediagnostik der Goldstandard sind. Als Ergänzung werden *in* vitro-Tests eingesetzt, mit denen die Anwesenheit von spezifischen IgE-Antikörpern gegen ein oder mehr als ein definiertes Allergen nachgewiesen wird.

Für einen solchen Nachweis werden Allergene an Matrizes wie Schwämmchen, Membranen oder Kügelchen gebunden und mit Patientenseren kontaktiert. Anschließend wird die Menge an gebundenem IgE mit Hilfe eines markierten anti-human-lgE Antikörpers bestimmt.

Die Vorteile der spezifischen IgE-Bestimmung *in vitro* umfassen ihre leichte Durchführbarkeit, Standardisierbarkeit und Aufskalierbarkeit. Allerdings kann man häufig nicht zwischen einer klinisch relevanten Allergie und einer bloßen Sensibilisierung unterscheiden.

Weitere Nachteile sind die schlechte Verfügbarkeit von manchen Antigenen und eine mögliche Konformationsänderung des Allergens durch dessen Kopplung an eine Matrix, was die Affinität zum nachzuweisenden IgE-Antikörper verändern kann. Allergenextrakte können in vielen Fällen nur unter Schwierigkeiten oder gar nicht standardisiert werden. Kleinere Allergene müssen zudem an Carriermoleküle wie z. B. Serumalbumin gebunden werden. Weiterhin muss die Spezifität für Allergene von IgE-Antikörpern in Serum und Membran gebundenen IgE-Antikörper auf Effektorzellen wie Basophilen und Mastzellen nicht übereinstimmen, da nur ein langsamer Austausch von gelöstem und gebundenem IgE stattfindet. Schließlich bestimmen zusätzliche Faktoren, die bei einem *In vitro-Test* zum Nachweis spezifischer IgE-Antikörper nicht abgebildet werden können, ob tatsächlich eine zelluläre allergische Reaktion ausgelöst wird, beispielsweise das Gesamtverhältnis von gesamt-lgE zu spezifischem IgE im Blut oder die Affinität der Antikörper.

Insbesondere dann, wenn IgE- und Hauttests unklare Ergebnisse liefern, ist daher empfehlenswert, als Ergänzung ein zelluläres Testsystem heranzuziehen wie den Basophilen-Degranulationstests oder den Basophilen-Aktivierungstest (BAT). Üblicherweise wird der Begriff "BAT" mit Bezug auf Durchflusszytometrie-Tests gebraucht.

Der besondere Vorteil der zellulären Tests gegenüber der spezifischen IgE-Antikörperbestimmung, die lediglich den gelösten, biologisch nicht aktiven IgE-Anteil im Serum bestimmt, besteht darin, dass sie die tatsächliche zellbiologische Reaktion erfassen, welche durch die Bindung des Allergens an Rezeptor-gebundene IgE-Antikörper an der Zelloberfläche der Basophilen und deren Quervernetzung ausgelöst wird. Die zellulären Tests ermöglichen es außerdem, die Schwelle der Degranulationsfähigkeit zu bestimmen. Dazu besteht insbesondere nach einer Immuntherapie zur Überwachung des Therapieerfolges des Patienten Bedarf.

Grundsätzlich werden zelluläre Tests in der Weise durchgeführt, dass zunächst Patientenblut mit einem Allergen kontaktiert wird. War der Patient bereits zuvor sensibilisiert, so kommt es zur Bindung des Allergenes an Rezeptor-gebundene allergenspezifische IgE-Antikörper, die an der Zelloberfläche der Basophilen lokalisiert sind. Dies führt zu einer Kreuzvernetzung der IgE-Rezeptoren, die wiederum eine Degranulation der Basophilen Granulozyten und damit die Freisetzung von Mediatoren aus intrazellulären Granula auslöst. Diese Mediatoren, z. B. Histamin, Leukotriene und Tryptase, können mit Hilfe von Enzymimmunoassays im Überstand quantifiziert werden. Die Ausschüttung der Mediatoren geht mit einer erhöhten Konzentration an der Zelloberfläche von Markern wie CD63 oder CD203c einher, welche zuvor auf den Membranen der intrazellulären Granula lokalisiert waren und somit ebenfalls eine zelluläre Reaktion zeigen. Die Ausschüttung der Mediatoren zeigt damit eine zelluläre Reaktion auf das Allergen.

Diese verstärkte Oberflächenkonzentration wird bislang in der Routinediagnostik durchflusszytometrisch mit Hilfe von Antikörpern gegen die aktivierungsspezifischen Zelloberflächenmarker nachgewiesen, die an einem Fluorophor gekoppelt sind. Die zu untersuchenden Zellen fließen dabei einzeln hintereinander in einem Flüssigkeitsstrahl durch eine dünne Messkammer und passieren mehrere Laserstrahlen verschiedener Wellenlänge, wobei ein für jeden Zelltyp charakteristisches Streulicht erzeugt wird und gleichzeitig, in Abhängigkeit der jeweiligen Wellenlänge, die an die Antikörper gekoppelten Fluorophore angeregt werden, sofern aktivierte Zellen vorhandeln sind. Das vom angeregten Fluorophor emittierte Licht und das Streulicht werden von Detektoren erfasst.

Nachteile dieses Testsystems sind die hohen Anschaffungskosten für ein Durchflusszytometer, die Komplexität der Testergebnisse, die nur von speziell geschultem Fachpersonal interpretiert werden können sowie die schwierige Standardisierbarkeit. Zudem kann mit einem Gerät immer nur eine Probe gleichzeitig gemessen werden.

Ein weiteres der Erfindung zugrunde liegendes Problem betrifft die Qualität von Proben, die aktivierbare Basophile umfassen. Die Qualität der Proben und damit deren Eignung für diagnostische Verfahren hängt davon ab, ob Gewinnung, Transport und Lagerung fachmännisch durchgeführt wurden. Beispielsweise kann eine Exponierung der Proben gegenüber Hitze oder ungeeigneten Puffern zum Absterben der Zellen führen.

Ist die Qualität unzureichend, so kann es zu falsch-negativen Ergebnissen kommen, d.h. ein negatives Ergebnis ist nicht dadurch bedingt, dass der Patient nicht allergisch ist, sondern dadurch, dass die Probe von ihm von unzureichender Qualität ist, genauer keine oder zu wenig aktivierbare Basophile enthält.

Es ist besonders ineffizient, wenn die mangelnde Qualität einer Probe erst bei der Durchführung eines aufwändigen Verfahrens wie der Durchflusszytometrie festgestellt wird.

Es besteht daher Bedarf an einem Verfahren und dazu brauchbaren, die Qualität einer Probe effizient, d.h. in einem für Hochdurchsatz tauglichen Verfahren festzustellen. Ein solches Verfahren wird zwar nicht mit einem Allergen durchgeführt und liefert daher kein diagnostisch nützliches Ergebnis, lässt aber eine Vorhersage zu, ob die Probenqualität für ein in einem getrennten Ansatz durchgeführtes diagnostisches Verfahren geeignet ist. Dieses Verfahren kann zuvor, nachträglich oder parallel zum diagnostischen Verfahren durchgeführt werden. Dieses Verfahren oder die dabei eingesetzten Reagenzien können dazu dienen, die Anzahl oder den Anteil aktivierbarer Basophiler in einer Zellpräparation, bevorzugt einer Probe zu bestimmen.

DE10235310 offenbart einen Test, bei dem die Aktivierung von Basophilen mit Hilfe eines fluoreszierenden Calciumfarbstoffes nachgewiesen wird. Es wird über eine Dichtegradientenzentrifugation fraktioniertes Blut eingesetzt.

WO2012/044756, US2009/0246805, WO98/32014 und EP2037269 offenbaren durchflusszytometrische Tests zum Nachweis der Aktivierung von Basophilen.

Von diesem Hintergrund besteht eine der vorliegenden Erfindung zugrundeliegende Aufgabe darin, ein Verfahren und dazu geeignete Reagenzien bereitzustellen, mit dem bzw. mit denen die oben genannten Nachteile von im Stand der Technik beschriebenen Verfahren überwunden werden können, insbesondere ein Basophilen-Aktivierungstest, der mit einem anderen System als einem Durchflusszytometer durchgeführt werden kann.

Insbesondere besteht eine Aufgabe darin, ein System zu schaffen, dass die *gleichzeitige* Abarbeitung einer Vielzahl von Proben in einem Hochdurchsatzverfahren erlaubt. Weiterhin sind Reproduzierbarkeit und Standardisierbarkeit zu gewährleisten.

Diese und weiteren Aufgaben werden durch den Gegenstand der vorliegenden Erfindung insbesondere durch den Gegenstand der beigefügten unabhängigen Ansprüche gelöst, wobei sich bevorzugte Ausführungsformen aus den Unteransprüchen ergeben.

Hiermit wird ein Verfahren zur Diagnose einer Allergie, bevorzugt ein für den Hochdurchsatz geeignetes Verfahren, offenbart, umfassend die Schritte:
a) Kontaktieren von Basophilen aus einer Vollblutprobe eines Patienten mit einem Allergen in einer wässrigen Lösung in Anwesenheit des Vollblutes mit einem Volumenanteil des Vollblutes an der wässrigen Lösung von wenigstens 20 Volumenprozent unter Bedingungen, die eine Aktivierung der Basophilen durch das Allergen zulassen,
b) Anreichern der Basophilen aus a) und
c) Nachweisen aktivierter Basophiler,
wobei das Nachweisen in Schritt c) dadurch erreicht wird, dass ein für aktivierte Basophile charakteristischer Marker mittels Chemilumineszenz detektiert wird.

In einem ersten Aspekt wird das die Erfindung zugrundeliegende Problem gelöst durch ein Verfahren zur Diagnose einer Allergie, umfassend die Schritte:
a) Kontaktieren von Basophilen aus einer Vollblutprobe eines Patienten mit einem Allergen in einer wässrigen Lösung in Anwesenheit des Vollblutes mit einem Volumenanteil des Vollblutes an der wässrigen Lösung von wenigstens 20 Volumenprozent unter Bedingungen, die eine Aktivierung der Basophilen durch das Allergen zulassen,
b) Anreichern der Basophilen aus a) und
c) Nachweisen aktivierter Basophiler,
   wobei das Nachweisen in Schritt c) dadurch erreicht wird, dass ein an der Oberfläche von Basophilen lokalisierter Marker mittels Chemilumineszenz detektiert wird,
   wobei in Schritt b) Basophile dadurch angereichert werden, dass sie an einen Liganden binden, der spezifisch an ein Polypeptid bindet, das ein für aktivierte Basophile charakteristischer Marker ist,
   und/oder das Nachweisen in Schritt c) dadurch erreicht wird, dass ein für aktivierte Basophile charakteristischer Marker mittels Chemilumineszenz detektiert wird.

In einer bevorzugten Ausführungsform handelt sich bei dem Vollblut um unprozessiertes Vollblut.

In einer weiteren bevorzugten Ausführungsform wird Schritt a) in einer wässrigen Lösung mit einem Vollblutanteil von wenigstens 30, bevorzugt 35, noch bevorzugter 40, noch bevorzugter 45 Volumenprozent durchgeführt.

In einer weiteren Ausführungsform umfasst das Verfahren den Schritt Stoppen der Aktivierung von Basophilen, bevorzugt vor Schritt b).

In einer weiteren Ausführungsform werden die Basophilen in Schritt b) dadurch angereichert, dass sie an einen Liganden binden, der spezifisch an ein Polypeptid bindet, das bei Basophilen an der Zelloberfläche lokalisiert ist. Bei diesem Polypeptid kann es sich um ein Polypeptid handeln, das unabhängig von ihrem Aktivierungszustand an der Zelloberfläche der Basophilen lokalisiert ist, und/oder um einen für aktivierte Basophile charakteristischen Marker, bevorzugt einen *weiteren* für aktivierte Basophile charakteristischen Marker, der sich von dem Marker unterscheidet, der erfindungsgemäß mittels Chemilumineszenz detektiert wird. In einer besonders bevorzugten Ausführungsform handelt es sich um ein Polypeptid, das unabhängig vom Aktivierungszustand der Basophilen an ihrer Zelloberfläche lokalisiert ist.

In einer weiteren Ausführungsform ist der Ligand, der spezifisch an ein Polypeptid bindet, das bei Basophilen unabhängig von ihrem Aktivierungszustand an ihrer Zelloberfläche lokalisiert ist, ein monoklonaler Antikörper gegen das Polypeptid.

In einer bevorzugten Ausführungsform werden die Basophilen in Schritt b) immobilisiert.

In einer bevorzugten Ausführungsform werden die Basophilen in Schritt b) an einem Bead immobilisiert, bevorzugt einem magnetischen Bead.

In einer bevorzugten Ausführungsform werden die Basophilen nach dem Immobilisieren gewaschen.

In einer bevorzugten Ausführungsform werden die Erythrozyten in Schritt b) lysiert und entfernt.

In einer bevorzugten Ausführungsform werden die Erythrozyten vor dem Immobilisieren der Basophilen durch Zentrifugieren entfernt.

In einer bevorzugten Ausführungsform wird das Nachweisen in Schritt c) mit Hilfe eines mit einer chemilumineszenzfähigen Markierung versehenen Liganden gegen den an der Oberfläche von Basophilen lokalisierten Marker durchgeführt, bevorzugt einen Marker, der für aktivierte Basophile charakteristisch ist.

In einer bevorzugten Ausführungsform handelt es sich bei dem mit einer chemilumineszenzfähigen Markierung versehenen Liganden um einen monoklonalen Antikörper, der selbst eine chemilumineszenzfähige Markierung aufweist oder an einen sekundären Antikörper mit einer chemilumineszenzfähigen Markierung bindet.

In einer bevorzugten Ausführungsform werden die Schritte a), b) und c) in einer Mikrotiterplatte ausgeführt.

In einer bevorzugten Ausführungsform wird Schritt a) vor den Schritten b) und c) durchgeführt.

In einem weiteren Aspekt wird die der Erfindung zugrundeliegende Aufgabe gelöst durch ein Kit gemäß Anspruch 16, nämlich ein Kit umfassend ein Gefäß zur Kontaktierung der Basophilen mit dem Allergen, wobei es sich bei dem Gefäß bevorzugt um eine Mikrotiterplatte handelt, optional ein Allergen, einen Liganden, der spezifisch gegen ein an der Zelloberfläche von Basophilen lokalisiertes Polypeptid bindet, zur Anreicherung von Basophilen und einen Liganden gegen einen an der Oberfläche von Basophilen lokalisierten Marker zur Detektion, wobei der Ligand, der spezifisch gegen den an der Oberfläche von Basophilen lokalisierten Marker bindet, zur Detektion eine chemilumineszenzfähige Markierung aufweist, wobei der Ligand, der spezifisch gegen ein an der Zelloberfläche von Basophilen lokalisiertes Polypeptid bindet, an einem festen Träger immobilisiert ist, wobei es sich bei der chemilumineszenzfähigen Markierung um ein Enzym handeln kann, das eine Chemilumineszenz-Reaktion katalysiert, oder um ein Molekül, das selbst unter geeigneten Bedingungen ein Chemilumineszenz-Signal erzeugt, wobei das Enzym im ersten Falle in Kombination mit einer chemilumineszenzfähigen Substratlösung im Kit enthalten ist, und wobei das an der Zelloberfläche von Basophilen lokalisierte Polypeptid und/oder der an der Oberfläche von Basophilen lokalisierte Marker charakteristisch für aktivierte Basophile ist, optional ein Mittel zum Stoppen der Aktivierung der Basophilen, optional ein Mittel zum Lysieren und/oder Entfernen von Erythrozyten. Bei dem erfindungsgemäßen mit einer chemilumineszenzfähigen Markierung versehenen Liganden gegen den Marker handelt es sich bevorzugt um einen monoklonalen Antikörper. Bei dem immobilisierten Liganden, der an ein unabhängig von ihrem Aktivierungszustand an der Zelloberfläche von Basophilen lokalisiertes Polypeptid bindet, handelt es sich bevorzugt um einen monoklonalen Antikörper gegen das Polypeptid.

Die in einem erfindungsgemäßen Kit enthaltenen Reagenzien können auch mit einem oder ohne ein Gefäß zur Kontaktierung der Basophilen mit dem Allergen zur Herstellung eines Kits zur Diagnose einer Allergie verwendet werden.

Das Problem wird in einem weiteren Aspekt gelöst durch eine Verwendung gemäß Anspruch 17, nämlich eine Verwendung eines Liganden, der spezifisch gegen ein an der Zelloberfläche von Basophilen lokalisiertes Polypeptid bindet,zur Anreicherung von Basophilen zusammen mit einem Liganden, der gegen einen an der Oberfläche von Basophilen lokalisierten Marker zur Detektion bindet, zur Diagnose einer Allergie oder zur Herstellung eines Kits zur Diagnose einer Allergie oder zum Screening von Kandidatenwirkstoffen für die Therapie einer Allergie gegen ein Allergen, wobei der Ligand gegen den an der Oberfläche von Basophilen lokalisierten Marker zur Detektion eine chemilumineszenzfähige Markierung aufweist, wobei es sich bei der chemilumineszenzfähigen Markierung um ein Enzym handeln kann, das eine Chemilumineszenz-Reaktion katalysiert, oder um ein Molekül, das selbst unter geeigneten Bedingungen ein Chemilumineszenz-Signal erzeugt, wobei das Enzym im ersten Falle in Kombination mit einer chemilumineszenzfähigen Substratlösung verwandt wird, wobei der Ligand, der spezifisch gegen ein an der Zelloberfläche von Basophilen lokalisiertes Polypeptid bindet, an einem festen Träger immobilisiert ist, und wobei das an der Zelloberfläche von Basophilen lokalisiertes Polypeptid und/oder der an der Oberfläche von Basophilen lokalisierte Marker charakteristisch für aktivierte Basophile ist.

In einer bevorzugten Ausführungsform ist die Verwendung zur Erhöhung der Sensitivität, bevorzugt in einem Hochdurchsatzverfahren.

Die vorliegende Erfindung betrifft ein Verfahren zur Diagnose einer Allergie zunächst umfassend den Schritt a) Kontaktieren von Basophilen aus einer Vollblutrobe eines Patienten mit einem Allergen in einer wässrigen Lösung. Bei der Vollblutprobe handelt es sich bevorzugt um Vollblut mit antikoagulierend wirkenden Substanzen. Das Vollblut wird der wässrigen Lösung vor dem eigentlich Kontaktieren der Basophilen mit dem Allergen in einer Menge hinzugefügt, dass sein finaler Volumenanteil an der wässrigen Lösung wenigstens 20, bevorzugter 25, 30, 35, 40, 45, 50, 55, 60, 65, 70, 75 oder 80 Volumenprozent beträgt. Die Anwesenheit des Vollblutes führt dazu, dass das Kontaktieren der Basophilen mit dem Allergen unter nahezu physiologischen Bedingungen abläuft.

Bei dem Verfahren handelt es sich bevorzugt um ein für den Hochdurchsatz geeignetes Verfahren. Darunter wird in einer bevorzugten Ausführungsform ein Verfahren verstanden, das die parallele Abarbeitung einer Vielzahl von Proben, beispielsweise wenigstens 2, 4, 8, 16 oder 32 Proben gestattet, wobei die Verfahrensschritte, die wenigstens 50, bevorzugter 60, 70, 80 oder 90% der Abarbeitungszeit für eine einzelne Probe erfordern, im Wesentlichen gleichzeitig mit den Proben von statten gehen können. Verfahren, bei denen der eigentliche Messvorgang zwar mittels einer Messvorrichtung bei allen Proben nacheinander durchgeführt wird, die individuelle, nicht-parallele Abarbeitung einer Probe aber nicht länger als 5, bevorzugter 4, 3, 2, 1 Minute oder 30 Sekunden nicht überschreitet, sind umfasst. Für den Hochdurchsatz geeignete Verfahren umfassen beispielsweise die Chemilumineszenzmessung in dafür geeigneten Mikrotiterplatten. Hingegen ist beispielsweise die Durchflusszytometrie kein für den Hochdurchsatz geeignetes Verfahren.

Bei dem Vollblut handelt es sich bevorzugt um unprozessiertes Vollblut. In einer bevorzugten Ausführungsform wird unter dem Begriff "unprozessiertes Vollblut", wie hierin verwendet, verstanden, dass das Blut zwar verdünnt sein kann oder ihm chemische Substanzen zur Konservierung zugesetzt werden, bevorzugt antikoagulierend wirkende Substanzen wie Heparin, dass es jedoch nicht fraktioniert ist, beispielsweise durch Zentrifugation wie eine Dichtegradientenzentrifugation.

Schritt a) kann auch mit mehr als einem Allergen und/oder einem Allergen in mehr als einer Konzentration in unterschiedlichen, parallel abgearbeiteten Ansätzen durchgeführt werden. Insbesondere kann das Antigen in zwei oder mehr, bevorzugt drei oder mehr unterschiedlichen Konzentrationen in einer entsprechenden Zahl von parallelen Ansätzen eingesetzt werden. Bevorzugt werden zusätzlich eine Positivkontrolle und eine Negativkontrolle mitgeführt. In einer bevorzugten Ausführungsform handelt es sich bei dem Allergen um ein Material ausgewählt aus der Gruppe umfassend Milbenkulturen und Milbenfäkalien der taxonomischen Hauptgruppe Animalia Arthropoda; Schuppen, Haar, Speichel, Kot oder anderen Sekrete, die von der taxonomischen Hauptgruppe Animalia Chordata stammen; Sporen oder Partikel, die von der taxonomischen Hauptgruppe Funghi Ascomycetes stammen; Pollen, der von der taxonomischen Hauptgruppe Corniferopsida stammt, Pollen, der von der taxonomischen Hauptgruppe Plantae Magnoliopsidae stammt, Pollen, der von der taxonomischen Hauptgruppe Plantae Liliopsidae stammt; Gift oder Sekrete, die von der taxonomischen Hauptgruppe Animalia Arthropoda stammen; Kautschuk oder Produkte, die einen solchen Kautschuk umfassen, der von Bäumen der taxonomischen Hauptgruppe Plantae Magnoliopsidae stammt. In einer bevorzugten Ausführungsform handelt es sich bei dem Allergen um eine Arzneimittelsubstanz ausgewählt aus der Gruppe umfassend Antibiotika, bevorzugter β-Lactame, noch bevorzugter aus der Gruppe umfassend Penicillin G, Penicillin V, PPL, MDM, Amoxicillin und Ampicillin; Cephalosporine, noch bevorzugter aus der Gruppe umfassend Cefazolin, Cefamandol, Cefaclor, Cefonaxim, Ceftazidim, Cefotaxim, Ceftazidim, Cefepim;, Carbapeneme, Monobactame, β-Lactamase-Inhibitoren, noch bevorzugter Clavulansäure; Makrolide, Aminoglycoside, Rifamycine, Glykopetide, Polypeptide, Tetracycline, Imidazole, Chinolone, Pyrazolone, , noch bevorzugter Sulfomethoxazol; Streptogramine, Nitrofurane, Isoniazide, Pentamidine; Antiseptika, bevorzugtervorzugsweise Chlorhexidin, Fungizide, Antivirale Mittel, Anti-Malaria-Mittel, Analgetika, COX-2-Inhibitoren und nicht-steroidale entzündungshemmende Arzneimittel, bevorzugt aus der Gruppe umfassend Aspirin, Lys-Aspirin, luprofen, Ketoprofen, Diclofenac, Naproxen, Paracetamol, Dipyron, Indomethacin, Mefenaminsäure, Phenylbutazon und Propyphenazon; Neuromuskuläre Blocker, bevorzugt aus der Gruppe umfassend Suxamethonium, Atracurium, Cis-Atracurium, Mivacurium, Pancuronium, Rocuronium, Vecuronium und Succinylcholin; Hypnotika und Lokalanästhetika, bevorzugt aus der Gruppe umfassendMidazolam, Propofol, Thiopental, Fentanyl und Lidocain; Tranquilizer; Opioide; Radio-Kontrastmittel, bevorzugt aus der Gruppe umfassend ionische lodkontrastmittel, nichtionische iodierte Kontrastmittel, Isosulfanblau, Patentblau und Methylenblau; Protonenpumpeninhibitoren. Antikonvulsiva und Neuroleptika, bevorzugt aus der Gruppe umfassend Carbamazepin, Phenytoin und Valproinsäure; Anti-psychotische Mittel; Antidepressiva; Dopamine; Anti-histamine; Kortikosteroide und Glukokortikoide; Chemotherapeutische und immunsuppressive Mittel; Diuretika; Antikoagulantien; Vasokonstriktoren und Vasodilatatoren; Herzarzneimittel, bevorzugt aus der Gruppe umfassendStatine, ACE-Inhibitoren, Alpha-Rezeptorblocker, Beta-Rezeptorblocker Calciumantagonisten und Anti-Hypertonika; (Anti-)Geschwür Drogen; (Anti-)Schilddrüsenmittel; Östrogene; Heparine und Derivate; Insuline; Streptokinasen und Urokinasen;

In einer bevorzugten Ausführungsform handelt es sich bei dem Allergen um einen Kolloid-, Plasmaexpander oder Hilfsmittel, bevorzugt ausgewählt aus der Gruppe umfassend Albumin, Dextran, Gelatine, Hetastarch, Pentastarch, Sinistrin, Polydocanol 600 (Ethoxysclerol), Laktose, Carboxymethylcellulose, Hydroxypropylcellulose, Protamin und Aprotinin.

In einer bevorzugten Ausführungsform handelt es sich bei dem Allergen um einen Nahrungsmittelzusatzstoff, bevorzug ausgewählt aus der Gruppe umfassend Lebensmittelkonservierungsmittel, Lebensmittelfarbstoffe, Nahrungsmittelveredelungsmitte, Antioxidationsmittel und Emulgatoren.

In einer bevorzugten Ausführungsform handelt es sich bei dem Allergen um ein Umwelt- oder Schadstoffmittel, bevorzugt ausgewählt aus der Gruppe umfassend Isocyanate, Isothiazolinone, Formaldehyd, Ethylenoxid, Phthalsäureanhydrid, Chloramin T, DMSO, Latex und Enzyme, die in der Back-, Lebensmittelverarbeitung und Waschindustrie verwendet werden.

In einer bevorzugten Ausführungsform handelt es sich bei dem Allergen um ein Material ausgewählt aus der Gruppe umfassend eine Kultur und/oder Fäkalien einer Milbe, die ausgewählt ist aus der Gruppe bestehend aus Milben der Gattung Acarus, Glycyphagus, Lepidoglyphus, Tryophagus, Blomia, Dermatophagoides, Euroglyphus, Blatella und Periplaneta; Schuppen, Haar, Speichel oder Kot eines Tiers einer Gattung ausgewählt aus Canis, Felis oder Equus; Hefe, Schimmelpilz oder Pilze einer Gattung ausgewählt aus der Gruppe bestehend aus Cladosporium, Aspergillus, Penicillium, Alternaria und Candida; Pollen einer Gattung ausgewählt aus der Gruppe bestehend aus Chamaecyparis, Cryptomeria, Cupressus, Juniperus, Anthoxanthum, Cynodon, Dactylis, Festuca, Holcus, Hordeum, Lolium, Oryza, Paspalum, Phalaris, Phleum, Poa, Secale, Sorghum, Triticum, Zea, Ambrosia, Artemisia, Alnus, Betula, Corylus, Fraxinus, Olea und Platanus; Gift eines Insekts einer Gattung ausgewählt aus der Gruppe bestehend aus Apis Bombus, Dolichovespula, Polistes, Polybia, Vespa und Vespula; Kautschuk oder Produkte, die einen solchen Kautschuk umfassen, der aus der Gattung Hevea stammt.

In einer bevorzugten Ausführungsform handelt es sich bei dem Allergen um ein Material ausgewählt aus der Gruppe umfassend Shrimps oder eine Shrimps-haltigen Nahrung der taxonomischen Hauptgruppe Animalia Arthropoda; Hummer oder eine Hummer-haltigen Nahrung der taxonomischen Hauptgruppe Animalia Arthropoda; Früchte, Hülsenfrüchte, Getreide oder Bohnen, die aus der taxonomischen Hauptgruppe Plantae Liliopsidae stammen oder ein Nahrungsmittelprodukt, das solche Früchte, Hülsenfrüchte, Getreide und/oder Bohnen umfasst; Früchte, Hülsenfrüchte, Getreide oder Bohnen, die aus der taxonomischen Hauptgruppe Plantae Magnoliopsidae stammen oder ein Nahrungsmittelprodukt, das solche Früchte, Hülsenfrüchte, Getreide und/oder Bohnen umfasst; Nuss oder einer nusshaltigen Nahrung der taxonomischen Hauptgruppe Plantae Magnoliopsidae.

In einer bevorzugten Ausführungsform handelt es sich bei dem Allergen um Material ausgewählt aus der Gruppe umfassend Kuhmilch, Kuhmilch-haltiger Nahrung, Hühnereiweiß, Hühnereiweißhaltiger Nahrung, Fisch oder fischhaltige Nahrung.

In einer bevorzugten Ausführungsform handelt es sich bei dem Allergen um Material ausgewählt aus der Gruppe umfassend Hordeum, Oryza, Secale, Triticum, Zea, Arachis, Corylis, Juglans, Prunus, Anacardium, Pistacia und Glycine.

In einer bevorzugten Ausführungsform handelt es sich bei dem Allergen um ein Polypeptid umfassend ein Antigen oder eine Variante davon aus der Gruppe umfassen Aca s 13, Gly d 2, Lep d 2, Lep d 5, Lep d 7, Lep d 10, Lep d 13, Tyr p 2, Tyr p 3, Tyr p 10, Tyr p 13, Tyr p 24, Blo t 1, Blo t 2 Blo t 3, Blo t 4, Blo t 5, Blo t 6, Blo t 10, Blo t 11, Blo t 12, Blo t 13, Blo t 19, Blo t 21, Der f 1, Der f 2, Der f 3, Der f 6, Der f 7, Der f 10, Der f 11, Der f 13, Der f 14, Der f 15, Der f 16, Der f 17, Der f 18, Der f 22, Der m 1, Der p 1, Der p 2, Der p 3, Der p 4, Der p 5, Der p 6 Der p 7, Der p 8, Der p 9, Der p 10, Der p 11, Der p 14, Der p 20, Der p 21, Der p 23, Eur m 1, Eur m 2, Eur m 3, Eur m 4, Eur m 14, Bla g 1, Bla g 2, Bla g 4, Bla g 5, Bla g 6, Bla g 7, Bla g 8, Per a 1, Per a 3, Per a 6, Per a 7, Per a 9, Per a 10, Can f 1, Can f 2, Can f 3, Can f 4, Can f 5, Can f 6, Fel d 1, Fel d 2, Fel d 3, Fel d 4, Fel d 5w, Fel d 6w, Fel d 7, Fel d 8, Equ c 1, Equ c 2, Equ c 3, Equ c 4 and Equ c 5, Cla c 9m, Cla c 14, Cla h 2, Cla h 5, Cla h 6, Cla h 7, Ca h 8, Cla h 9, Cla h 10, Cla h 12, Asp fl 13, Asp f 1, Asp f 2, Asp f 3, Asp f 4, Asp f 5, Asp f 6, Asp f 7, Asp f 8, Asp f9, Asp f 10, Asp f 11, Asp f 12, Asp f 13, Asp f 15, Asp f 16, Asp f 17, Asp f 18, Asp f 22, Asp f 23, Asp f 27, A5p f 28, A5p f 29, Asp f 34, Asp n 14, Asp n 18, Asp n 25, Asp o 13, Asp o 21, Pen b 13, Pen b 26, Pen ch 13, Pen ch 18, Pen ch 20, Pen ch 31, Pen ch 33, Pen ch 35, Pen c 3, Pen c 13, Pen c 19, Pen c 22, Pen c 24, Pen C 30, Pen c 32, Pen o 18, Fus c 1, Fus c 2, Alt a 1, Alt a 3, Alt a 4, Alt a 5, Alt a 6, Alt a 7, Alt a 8, Alt a 10, Alt a 12, Alt a 13, Cand a 1, Cand a 3 and Cand b 2, Cha o 1, Cha o 2, Cup a 1, Cup s 1, Cup s 3, Jun a 1, Jun a 2, Jun a 3, Jun o 4, Jun s 1, Jun v 1 and Jun v 3, Ant o 1, Cyn d 1, Cyn d 7, Cyn d 12, Cyn d 15, Cyn d 22w, Cyn d 23, Cyn d 24, Dac g 1, Dac g 2, Dac g 3, Dac g 4, Dac g 5, Fes p 4, Hol I 1, Hol I 5, Hor v 1, Hor v 5, Lol p 1, Lol p 2, Lol p 3, Lol p 4, Lol p 5, Lol p 11, Pas n 1, Pha a 1, Pha a 5, Phl p 1, Phl p 2, Phl p 4, Phl p 5, Phl p 6, Phl p 7, Phl p 11, Phl p 12, Phl p 13, Poa p 1, Poa p 5, Sec c 1, Sec C 5, Sec c 20, Sor h 1, Tri a 15, Tri a 21, Tri a 27, Tri a 28, Tri a 29, Tri a 30, Tri a 31, Tri a 32, Tri a 33, Tri a 34, Tri a 35, Zea m 1, Zea m 12, Amb a 1, Amb a 2, Amb a 3, Amb a 4, Amb a 5, Amb a 6, Amb a 7, Amb a 8, Amb a 9 and Amb a 10, Amb p 5, Amb t 5, Art v 1, Art v 2, Art v 3, Art v 4, Art v 5, Art v 6, Aln g 1, Aln g 4, Bet v 1, Bet v 2, Bet v 3, Bet v 4, Bet v 6, Bet v 7, Cor a 10, Fra e 1, Ole e 1, Ole e 2, Ole e 3, Ole e 4, Ole e 5, Ole e 6, Ole e 7, Ole e 8, Ole e 9, Ole e 10, Ole e 11, Pla a 1, Pla a 2, Pla a 3, Pla or 1, Pla or 2 and Pla or 3, Api c 1, Api d 1, Api m 1, Api m 2, Api m 3, Api m 4, Api m 5, Api m 6, Api m 7, Api m 8, Api m 9, Api m 10, Api m 11, Bom p 1, Bom p 4, Bom t 1, Bom t 4, Dol a 5, Dol m 1, Dol m 2, Dol m 5, Pol a 1, Pol a 2 and Pol a 5, Pol d 1, Pol d 4, Pol d 5, Pol e 1, Pol e 4 and Pol e 5, Pol f 5, Pol g 1, Pol g 5, Poly p 1, Poly s 5, Vesp c 1, Vesp c 5, Vesp ma 2, Vesp ma 5, Vesp m 1, Vesp m 5, Ves g 5, Ves m 1, Ves m 2, Ves m 5, Ves p 5, Ves s 1, Ves s 5, Ves vi 5, Ves v 1, Ves v 2, Ves v 3, Ves v 5, Hev b 1, Hev b 2, Hev b 3, Hev b 4, Hev b, 5, Hev b 6, Hev b 7, Hev b 8, Hev b 9, Hev b 10, Hev b 11, Hev b 12, Hev b 13 and Hev b 14, Hor v 12, Horv 15, Horv 16, Hor v 17, Hor v 21, Ory s 12, Sec c 20, Tri a 12, Tri a 14, Tn a 18, Tri a 19, Tri a 21, Tri a 25, Tri a 26, Tri a 36, Zea m 14, Zea m 25, Ara h 1, Ara h 2, Ara h 3, Ara h 4, Ara h 5, Ara h 6, Ara h 7, Ara h 8, Ara h 9, Ara h 10, Ara h 11, Cor a 1, Cor a 2, Cor a 8, Cor a 9, Cor a 11, Cor a 12, Cor a 13, Cor a 14, Jug n 1, Jug n 2, Jug r 1, Jug r 2, Jug r 3, Jug r 4, Pru du 3, Pru du 4, Pru du 5, Pru du 6, Ana o 1, Ana o 2, Ana o 3, Pis v 1, Pis v 2, Pis v 3, Pis v 4, Pis v 5, Gly m 1, Gly m 2, Gly m 3, Gly m 4, Gly m 5 and Gly m 6.

Schritt a) kann in einem Gefäß zur Kontaktierung der Basophilen mit dem Allergen durchgeführt werden. In einer bevorzugten Ausführungsform handelt es sich bei dem Gefäß um eine Mikrotiterplatte. In einer weiteren bevorzugten Ausführungsform ist das Allergen oder sind die Allergene in dem Gefäß in lyophilisierter Form vorgelegt und gehen bei der Zugabe von wässriger Lösung und Vollblutprobe in die flüssige Phase über.

In einer bevorzugten Ausführungsform ist das Antigen Ara h 7 Isotyp 7.0201 (EP17000245). In einer weiteren bevorzugten Ausführungsform ist das Antigen das in EP16000165.7 beschriebene Antigen der Macadamia-Nuss. Auch modifizierte Polypeptide können als Antigen eingesetzt werden, beispielsweise die in der EP 15001277.1 offenbarten Oleosin-Konstrukte.

In einer bevorzugten Ausführungsform umfasst das Verfahren den Schritt Stoppen der Aktivierung der Basophilen, bevorzugt vor Schritt b). Dem Fachmann sind verschiedene Mittel zum Stoppen der Aktivierungsreaktion bekannt, beispielsweise die Zugabe von Calziumbindenden Agenzien wie EDTA, die Zugabe von Azid, bevorzugt Natriumazid oder die Kühlung der Probe, bevorzugt auf eine Temperatur von höchstens 4 °C.

Bevorzugt nach Schritt a) werden die Basophilen in Schritt b) angereichert. In einer bevorzugten Ausführungsform wird unter dem Begriff _{"}Anreichern", wie hierin verwendet, verstanden, dass die Anzahl der Basophilen relativ zur Anzahl der Gesamtheit aller Zellen oder zur Leukozytenanzahl, bevorzugt zur Leukozytenzahl in der Vollblutprobe erhöht wird, bevorzugt um den Faktor wenigstens 2, 5, 10, 20, 50, 100 oder 1000, bevorzugter wenigstens 1000. Dies geschieht bevorzugt dadurch, dass sie einen Liganden binden, der spezifisch über eine erste Bindungsstelle an ein Polypeptid bindet, das bei Basophilen abhängig oder unabhängig, bevorzugt unabhängig, von ihrem Aktivierungszustand exprimiert und an ihrer Zelloberfläche lokalisiert ist, während es bei anderen Zellen oder wenigstens der Mehrheit der anderen Zellen nur in geringem Maße oder bevorzugt gar nicht dort lokalisiert ist. Dieses Polypeptid muss also als Selektionsmittel geeignet sein, um Basophile relativ zu der Gesamtzahl der Zellen in einer Blutprobe anzureichern. Dabei ist es akzeptabel, wenn die Anreicherung nicht vollständig gelingt, sondern ein kleiner Teil der anderen Zellen in der Probe verbleibt, beispielsweise dendritische Zellen. Bevorzugt ist das Polypeptid aus der Gruppe von unabhängig vom Aktivierungszustand der Basophilen an der Zelloberfläche der Basophilen lokalisierten Polypeptiden ausgewählt, die CD123 (Datenbankcode NM_002183), CD193 (NP_001828), FcεRl (NM_002001), IgE, CD203c (NM_005021) und CRTH2 (NM_004778) umfasst, und ist bevorzugt CD123 oder IgE, noch bevorzugter CD123. Sämtliche in dieser Anmeldung verwandten Datenbankcodes beziehen sich auf die Sequenz, die bei der durch die Nummer festgelegten Datenbank zum Zeitpunkt des Anmeldetags der frühesten prioritätsbegründenden Anmeldung online zugänglich war. Der Ligand ist bevorzugt ein monoklonaler Antikörper gegen das Polypeptid, bevorzugt ein monoklonaler Antikörper gegen CD123 oder IgE, noch bevorzugter gegen CD123 (Agis, H., Füreder, W., Bankl, H. C., Kundi, M., Sperr, W. R., Willheim, M., ... & Valent, P. (1996). Comparative immunophenotypic analysis of human mast cells, blood basophils and monocytes. Immunology, 87(4), 535-543.; Sainte-Laudy, J. Sabba, A., Vallon C, Guerin JC (1998). Analysis of anti-lgE and allergen induced human basophil activation by flow cytometry. Comparison with histamine release. Inflamm Res. 47(10), 401-8.). In einer noch bevorzugteren Ausführungsform wird dabei ein isolierter monoklonaler Antikörper eingesetzt. In einer weiteren bevorzugten Ausführungsform binden die Basophilen an mehr als einen Liganden, der spezifisch über eine erste Bindungsstelle an ein Polypeptid bindet, das bei Basophilen abhängig oder unabhängig, bevorzugt unabhängig von ihrem Aktivierungszustand exprimiert und an ihrer Zelloberfläche lokalisiert ist, wobei das Polypeptid bevorzugt aus der Gruppe von unabhängig vom Aktivierungszustand der Basophilen an der Zelloberfläche der Basophilen lokalisierten Polypeptiden ausgewählt ist, die CD123 (Datenbankcode NM_002183), CD193 (NP_001828), FcεRl (NM_002001), IgE, CD203c (NM_005021) und CRTH2 (NM_004778) umfasst, bevorzugter zwei oder drei Liganden, wobei noch bevorzugter einer der Liganden CD123 ist. Ein Polypeptid, das bei Basophilen abhängig von ihrem Aktivierungszustand exprimiert und an ihrer Zelloberfläche lokalisiert ist, ist bevorzugt aus der Gruppe ausgewählt, die Basogranulin (Mochizuki A, McEuen AR, Buckley MG, Walls AF. The release of basogranulin in response to IgE-dependent and IgE-independent stimuli: validity of basogranulin measurement as an indicator of basophil activation. J. Allergy Clin. Immunol. 2003 Jul;112(1):102-8.)], 2D7 Antigen [C L Kepley, S S Craig and L B Schwartz. Identification and partial characterization of a unique marker for human basophils. J Immunol June 15, 1995, 154 (12) 6548-6555;), Avidin bindender Mediator (Joulia R, Valitutti S, Didier A, Espinosa E. Direct monitoring of basophil degranulation by using avidin-based probes. J Allergy Clin Immunol. 2017), CD13 (NM_001150), CD45 (NM_001150), CD63 (NP_001244318.1), CD107a (NM_00556), CD11b (NM_000632), CD62L (NM_000655), CD11c (XM_011545852), CD164 (NM_001142401), CD45 (NM_001267798) und CD203c (NM_005021) umfasst, und ist bevorzugt CD63 (Knol, E. F., Mul, F. P., Jansen, H., Calafat, J., & Roos, D. (1991). Monitoring human basophil activation via CD63 monoclonal antibody 435. Journal of Allergy and Clinical Immunology, 88(3), 328-338.).

Bevorzugt weist der Ligand eine zweite Bindungsstelle auf, die genutzt werden kann, um ihn an einem festen Träger zu immobilisieren, bevorzugt spezifisch, wenn der Träger eine komplementäre Bindungsfähigkeit aufweist, genauer, die Fähigkeit, spezifisch an den Liganden zu binden. Bevorzugt weist der Ligand ein Molekül mit einer Bindungsfähigkeit auf, das aus der Gruppe ausgewählt ist, die Biotin, Streptavidin, His Tag, GST, Avidin, Neutravidin, Protein A, Protein G, Protein L, S-Tag und MBP umfasst. Bei dem festen Träger handelt es sich bevorzugt um einen Träger aus der Gruppe, die ein Bead, bevorzugt ein magnetisches Bead, eine Mikrotiterplatte, ein Reaktionsgefäß aus Plastik und ein Reaktionsgefäß aus Glass umfasst. In einer besonders bevorzugten Ausführungsform handelt es sich bei dem Träger um einen Bead, bevorzugt einen magnetischen Bead, und die wässrige Lösung umfassend den Bead befindet sich in einem Well einer Mikrotiterplatte. In einer bevorzugten Ausführungsform wird die wässrige Lösung in Schritt a) und/oder Schritt b) kontinuierlich durchmischt, beispielsweise durch Rühren oder Schütteln.

In einer bevorzugten Ausführungsverfahren das Lysieren und anschließende Entfernen von Erythrozyten, bevorzugt in Schritt b). Dem Fachmann sind Mittel zum Lysieren von Erythrozyten bekannt, beispielsweise sind sie in der US4902613, US6143567 oder WO85/05640 beschrieben. Zum Beispiel kann eine hypotone Lyse mit Wasser alleine oder in Anwesenheit von Diethylenglykol, Formaldehyd und Zitronensäure durchgeführt werden (US4902613).

Das Entfernen der Erythrozyten erfolgt bevorzugt über Zentrifugation.

Die Aktivierung von Basophilen wird erfindungsgemäß dadurch nachgewiesen, dass die Oberflächenkonzentration eines für die Aktivierung charakteristischen Markers mittels Chemilumineszenz detektiert wird. Bevorzugt ist der Marker aus der Gruppe ausgewählt, die Basogranulin (Mochizuki A, McEuen AR, Buckley MG, Walls AF. The release of basogranulin in response to IgE-dependent and IgE-independent stimuli: validity of basogranulin measurement as an indicator of basophil activation. J. Allergy Clin. Immunol. 2003 Jul;112(1):102-8.)], 2D7 Antigen [C L Kepley, S S Craig and L B Schwartz. Identification and partial characterization of a unique marker for human basophils. J Immunol June 15, 1995, 154 (12) 6548-6555;), Avidin bindender Mediator (Joulia R, Valitutti S, Didier A, Espinosa E. Direct monitoring of basophil degranulation by using avidin-based probes. J Allergy Clin Immunol. 2017 ), CD13 (NM_001150), CD45 (NM_001150), CD63 (NP_001244318.1), CD107a (NM_00556), CD11b (NM_000632), CD62L (NM_000655), CD11c (XM_011545852), CD164 (NM_001142401), CD45 (NM_001267798) und CD203c (NM_005021) umfasst, und ist bevorzugt CD63 (Knol, E. F., Mul, F. P., Jansen, H., Calafat, J., & Roos, D. (1991). Monitoring human basophil activation via CD63 monoclonal antibody 435. Journal of Allergy and Clinical Immunology, 88(3), 328-338.). Es besteht die Möglichkeit, mehr als einen dieser Marker zu detektieren. Bevorzugt wird dazu ein Ligand gegen einen für aktivierte Basophile charakteristischen Marker, bevorzugt ein monoklonaler Antikörper, mit einer chemilumineszenzfähigen Markierung versehen. In einer noch bevorzugteren Ausführungsform wird dabei ein isolierter monoklonaler Antikörper eingesetzt.

Bei der chemilumineszenzfähigen Markierung kann es sich um ein Enzym handeln, das eine Chemilumineszenz-Reaktion katalysiert, oder um ein Molekül, das selbst unter geeigneten Bedingungen ein Chemilumineszenz-Signal erzeugt, bevorzugt um letzteres Molekül, noch bevorzugter um einen Acridiniumester oder Acriniumsulfonamid. In einem vor Schritt a) durchgeführten Schritt kann ein Ligand gegen einen Marker, der für aktivierte Basophile charakteristisch ist, mit einer chemilumineszenzfähigen Markierung gekoppelt werden. Beispielsweise kann der monoklonale Antikörper mit einem Acridiniumester (AE) versetzt werden. AE Verbindungen sind hochsensitive Markierungen, die bereits in automatisierten Assays für klinische Diagnostik eingesetzt werden (I Weeks, I Beheshti, F McCapra, A K Campbell, J S Woodhead. Acridinium esters as high specific activity labels in immunoassay. Clin Chem 29: 1474-1479 (1983). Die Kopplung der AE Verbindungen an Antikörper ist gut etabliert und wird meist über funktionelle Gruppen wie das N-hydroxysuccinimidyl (NHS) im Phenolring erreicht. Zu diesem Zweck wird der gereinigte Antikörper mit AE (z.B. NSP-SA-NHS, NSP-DMAE oder NSP-DMAE-NHS der Firma Heliosense) bei Raumtemperatur für bestimmte Zeit umgesetzt. Anschließend wird der markierte Antikörper über Säulen oder andere Reinigungsverfahren wie HPLC von nicht-umgesetztem freiem AE abgetrennt.

In einer bevorzugten Ausführungsform hat der Acridiniumester die Formel: 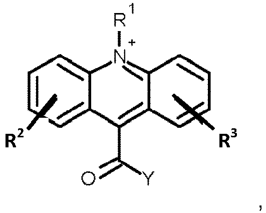 wobei R1 ein Alkyl, substituiertes Alkyl, Aryl, substituiertes Aryl und R2 und R3 unabhängig voneinander aus der Gruppe ausgewählt sind, die Wasserstoff, Alkyl, substituiertes Alkyl, Halogen, Cyanid, Hydroxy, Alkoxy, Thiol, Alkylmercapto umfasst, und wobei Y eine Abgangsgruppe, bevorzugt mit einem pKa<12 ist, bevorzugt aus der Gruppe umfassend Aryloxy, substituiertes Aryloxy, substituiertes Alkoxy, Mercapto, substituiertes Mercapto, Sulfonamidyl, substituiertes Sulfonamidyl, N-hydroxylamid, substituiertes N-hydroxylamid ausgewählt ist und wobei der Acridiniumester eine Gruppe enthält, über die er an ein Polypeptid, bevorzugt an einen Antikörper, gekoppelt werden kann bzw. gekoppelt ist, bevorzugt eine N-hydroxysuccinimidyl (NHS)-Gruppe. Geeignete Verbindungen und Verfahren sind in der WO2012/028167 beschrieben.

Für die Detektion kann ein Chemilumineszenz-Detektionskit (z.B. Firma Invitron) verwendet werden. Nach dem Waschen der ungebundenen Antikörper erfolgt im Luminometer nacheinander die automatische Zugabe der beiden Trigger (je 100µl; Trigger 1: enthält Hydrogenperoxid; Trigger 2: enthält Natriumhydroxid). Die Acridiniumester-Verbindung wird dabei unter alkalischen Bedingungen oxidiert, was Licht-emittierendes Acridon erzeugt. Das Signal wird in Form eines Lichtblitzes (Flash-Lumineszenz), welcher typischerweise 1-5 sec andauert, freigesetzt. Die kurze Dauer der Emission erfordert die Initiierung und Messung der Reaktion direkt am Detektor. Geeignete Luminometer ermöglichen die automatische Triggerzugabe und Detektion des Signals in der Mikrotiterplatte. Für eine höhere Intensität wir das gesamte Signal üblicherweise über das Messintervall integriert.

Eine Alternative ist die Kopplung des Antikörpers mit einem Enzym, bevorzugt ausgewählt aus der Gruppe umfassend Peroxidase, alkalische Phosphatase und β-Galaktosidase, welches ein chemilumineszenzfähiges Substrat umsetzen kann *(*Kricka L J. (2003).Clinical applications of chemiluminescence. Analytica chimica acta, 500(1): 279-286). Je nach Enzym wird dabei ein Substrat aus der Gruppe zyklische Diacylhydrazide, bevorzugt Luminol oder Isoluminol, Acridin, Dioxetan und ihre Derivate eingesetzt. Das am häufigsten eingesetzte Enzym dafür ist die Meerrettichperoxidase (HRP), die unter Anwesenheit von Wasserstoffperoxid die Oxidation von Luminol katalysiert. Das dabei freigesetzte Licht kann bei einer Wellenlänge von 425 nm detektiert werden. Eine mögliche Vorgehensweise ist in Neupert, W., Oelkers, R., Brune, K., Geisslinger, G. A new reliable chemiluminescence immunoassay (CLIA) for prostaglandin E2 using enhanced luminol as substrate. Prostaglandins. 1996 Nov;52(5):385-401 beschrieben.

Eine weitere Alternative ist die Verwendung von elektrogenerierter Chemilumineszenz. Tris(2,2-bipyridyI)ruthenium(II) wird bereits in einigen Immunoassays verwendet (Xiaoming Zhou, Debin Zhu, Yuhui Liao, Weipeng Liu, Hongxing Liu, Zhaokui Ma & Da Xing (2014). Synthesis, labeling and bioanalytical applications of a tris(2,2-bipyridyl)ruthenium(II)-based electrochemiluminescence probe. Nature Protocols Volume 9, Pages: 1146-1159). Nach Anlegen einer Spannung wird bei der Reaktion des Rutheniumkomplexes mit Tripropylamin (TPA) aus dem umgebenden Medium Chemilumineszenz mit einem Emissionsmaximum von 620 nm freigesetzt. Bevorzugt handelt es sich bei der chemilumineszenzfähigen Markierung dabei um einen chemilumineszenzfähigen Metallkomplex, bevorzugt umfassend Ruthenium.

In einer bevorzugten Ausführungsform katalysiert die chemilumineszenzfähigen Markierung die Emission durch eine andere Gruppe oder emittiert selbst bei einem Emissionsmaximum mit einer Wellenlänge von 400 nm oder mehr, bevorzugt 400 nm bis 650 nm, bevorzugter 400 nm bis 550 nm, noch bevorzugter 405 nm bis 500 nm, noch bevorzugter 405 nm bis 490 nm, noch bevorzugter 410 bis 490 nm, noch bevorzugter 410 bis 450 nm, am bevorzugtesten 415 bis 445 nm. Geeignete Verbindungen und Verfahren sind in Natrajan et al. (2010) Enhanced immunoassay sensitivity using chemiluminescent acridinium esters with increased light output, Analytical Biochemistry, 27 July 2010) beschrieben.

Bevorzugt werden eine Vielzahl von Reaktionen parallel in verschiedenen Wells in einer Mikrotiterplatte durchgeführt und die Chemilumineszenz-Signale mit Hilfe eines Plattenluminometers, wie er im Handel erhältlich ist (z.B. von Berthold Technologies) detektiert. Das Ausmaß der Aktivierung der Basophilen korreliert dabei mit der Intensität bzw. Zunahme des Chemilumineszenz-Signals relativ zu einer unstimulierten Kontrolle. Für eine positive Bewertung muss ein von der Testsubstanz abhängiger Schwellenwert überschritten werden, den der Fachmann routinemäßig festlegen kann.

Bevorzugt wird das Verfahren in der Reihenfolge ausgeführt, dass zunächst Schritt a), dann Schritt b) und schließlich Schritt c) ausgeführt wird. Es ist jedoch auch möglich, zunächst Schritt b) durchzuführen und anschließend Schritt a) und dann Schritt c). Alternativ können Schritt b) und Schritt c) gleichzeitig oder überlappend durchgeführt werden.

In einer besonders bevorzugten Ausführungsform wird nach Schritt a) zur wässrigen Lösung mit den Basophilen sowohl ein Ligand gegeben, der spezifisch an ein Polypeptid bindet, das bei Basophilen unabhängig von ihrem Aktivierungszustand an ihrer Zelloberfläche lokalisiert ist, als auch ein mit einer chemilumineszenzfähigen Markierung versehener Ligand gegen den Marker, der für aktivierte Basophile charakteristisch ist. Noch bevorzugter geschieht dies in Anwesenheit von einem Träger, wobei der Ligand der spezifisch an ein Polypeptid bindet, das bei Basophilen unabhängig von ihrem Aktivierungszustand an ihrer Zelloberfläche lokalisiert ist, noch bevorzugter an einem festen Träger wie einem Bead immobilisiert ist oder wird. Anschließend können die wässrige Lösung ausgetauscht und immobilisierte Basophile gewaschen werden, wobei überschüssige Liganden und nicht gebundene Zellen entfernt werden. Übrig bleiben, sofern vorhanden, Basophile, die über den Liganden, der spezifisch an ein Polypeptid bindet, das bei Basophilen unabhängig von ihrem Aktivierungszustand an ihrer Zelloberfläche lokalisiert wird, auf dem Träger immobilisiert sind. Von diesen immobilisierten Basophilen sind die durch die Kontaktierung mit dem Allergen tatsächlich aktivierten dadurch gekennzeichnet, dass an ihnen der Ligand gegen den Marker gebunden ist, der für aktivierte Basophile charakteristisch ist. Sofem letzterer Ligand nicht selbst mit einer chemilumineszenzfähigen Markierung versehen ist, kann, optional nach einem Waschschritt zur Entfernung von überschüssigen Liganden, in einem folgenden Schritt bei Bedarf ein sekundärer Antikörper mit einer chemilumineszenzfähigen Markierung hinzugegeben werden, der gegen den letztgenannten Liganden bindet, wobei dieser nur an den aktivierten Basophilen gebunden bleibt, nicht an nicht aktivierten. In einem weiteren Waschschritt kann überschüssiger sekundärer Antikörper entfernt werden.

Anschließend wird die Chemilumineszenz gemessen. Mit Hilfe dieses Verfahrens, insbesondere in Kombination mit der Entfernung von Erythrozyten, kann trotz des geringen Anteils der Basophilen beim Nachweis der Aktivierung eine Empfindlichkeit erreicht werden, die mit den Verfahren mittels Durchflusszytometrie vergleichbar ist.

Das erfindungsgemäße Verfahren und oder der erfindungsgemäße Kit oder ein oder mehr als ein Reagenz, wie es im erfindungsgemäßen Kit enthalten sein kann, kann auch zum Screening von Kandidatenwirkstoffen verwendet werden, um unter einer Auswahl von Kandidatenwirkstoffen therapeutisch wirksame Wirkstoffe zu identifizieren. In diesem Fall wird Schritt a) nach dem Kontaktieren der Basophilen mit einem Kandidatenwirkstoff durchgeführt. Im Falle eines therapeutisch wirksamen Kandidatenwirkstoffes ist relativ zu einem Ansatz ohne Kandidatenwirkstoff oder mit einem nicht wirksamen Kandidatenwirkstoff eine geringere Aktivierung festzustellen.

In einer bevorzugten Ausführungsform wird dabei unter dem Begriff "Kandidatenwirkstoff", wie hierein verwendet, eine Verbindung verstanden, von der angenommen wird bzw. die darauf untersucht werden soll, ob sie bei einem Patienten eine therapeutische Wirkung hat, bevorzugt in dem Sinne, dass sie allergische Reaktion lindert oder verhindert. In einer bevorzugten Ausführungsform wird ein oder mehr als ein bereits bekannter Wirkstoff darauf untersucht, der optional nur bei einer Untergruppe der unter der Allergie leidenden Patienten anschlägt, ob er bei einem bestimmten Patienten therapeutisch wirksam ist.

Die Erfindung betrifft die Verwendung des eine chemilumineszenzfähige Markierung aufweisenden Liganden gegen einen für aktivierte Basophile charakteristischen Liganden zur Diagnose einer Allergie oder zur Herstellung eines Kits zur Diagnose einer Allergie. Ebenfalls für die Diagnose einer Allergie kann das erfindungsgemäße Kit eingesetzt werden. Die Herstellung kann die Kopplung einer chemilumineszenzfähigen Markierung an dem Liganden umfassen. Der Ligand kann in einem Kit oder in einer Zusammensetzung oder sonstigen Kombination mit dem Liganden verwendet werden, der spezifisch an ein Polypeptid bindet, das bei Basophilen unabhängig von ihrem Aktivierungszustand an ihrer Zelloberfläche lokalisiert ist.

Ein erfindungsgemäßes Kit kann ein Reagenz enthalten, das Basophile unabhängig von der Anwesenheit eines Allergens aktiviert. Derartige Reagenzien sind im Stand der Technik beschrieben und umfassen, sind aber nicht beschränkt auf Formyl-methionyl-leucyl-phenylalanine (fMLP), Lonomycin, 12-Myristat-13-Acetat (PMA), fMLP und A23187, anti-human IgG, einen anti-lgE Rezeptor-Antikörper C40/80 und Sinomenine. Bevorzugt handelt es sich um fMLP und/oder um anti-Fc Epsilon-RI, noch bevorzugter fMLP.

Die vorliegende Erfindung betrifft weiterhin ein Verfahren und dafür geeignete Reagenzien, um die Qualität einer Probe effizient, d.h. in einem für Hochdurchsatz tauglichen Verfahren festzustellen. Ein solches Verfahren wird zwar nicht mit einem Allergen durchgeführt und liefert daher kein diagnostisch nützliches Ergebnis, lässt aber eine Vorhersage zu, ob die Probenqualität für ein in einem getrennten Ansatz durchgeführtes diagnostisches Verfahren geeignet ist.

Zu diesem Zweck wird das erfindungsgemäße Verfahren durchgeführt, wobei das Allergen durch ein Reagenz ersetzt wird, von dem bekannt ist, dass es aktivierbare Basophile unabhängig von der Anwesenheit eines Allergens aktiviert.

Als eine zusätzliche oder alternative Positivkontrolle kann statt der Probe des Patienten eine Basophilenpräparation in Kombination mit einem Allergen oder mit einem Reagenz eingesetzt werden, von dem bekannt ist, dass es aktivierbare Basophile unabhängig von der Anwesenheit eines Allergens aktiviert, wobei von der Basophilenpräparation bekannt ist, dass sie ausreichend reaktiv, d.h. durch Allergene aktivierbar ist. Sie kann allein oder in Kombination mit einem Allergen oder mit einem Reagenz eingesetzt werden, von dem bekannt ist, dass es aktivierbare Basophile unabhängig von der Anwesenheit eines Allergens aktiviert, in einem erfindungsgemäßen Kit enthalten sein.

Dieses Verfahren kann zuvor, nachträglich oder parallel zum diagnostischen Verfahren durchgeführt werden, oder unabhängig davon.

Die Erfindung betrifft weiterhin ein nicht-diagnostisches Verfahren zur Validierung oder zum Überprüfen der Zuverlässigkeit eines Verfahrens zum Nachweis einer Basophilenaktivierung oder zur Feststellung der Qualität der Probe, die Basophile enthält, bevorzugt die Aktivierbarkeit von in der Probe enthaltenden Basophilen, umfassend die Schritte:
a) Kontaktieren von Basophilen aus einer Vollblutprobe eines Patienten mit einem Reagenz, das Basophile unabhängig von der Anwesenheit eines Allergens aktiviert, unter Bedingungen, die eine Aktivierung der Basophilen durch das Allergen zulassen,
b) Anreichern der Basophilen aus a) und
c) Nachweisen aktivierter Basophiler,
wobei das Nachweisen in Schritt c) dadurch erreicht wird, dass ein für aktivierte Basophile charakteristischer Marker mittels Chemilumineszenz detektiert wird.

Bei dem Basophile unabhängig von der Anwesenheit eines Allergens aktivierenden Reagens kann es sich um formyl-methionyl-leucyl-phenylalanine (fMLP; z.B. von Sigma, St Louis, MO) und/oder um einen anti-lgE Rezeptor-Antikörper handeln.

Im Folgenden wird die Erfindung unter Bezugnahme auf die Figuren anhand von Ausführungsbeispielen erläutert. Die beschriebenen Ausführungsformen sind in jeder Hinsicht lediglich beispielhaft und nicht als einschränkend zu verstehen, und verschiedene Kombinationen der angeführten Merkmale sind vom Umfang der Erfindung umfasst.

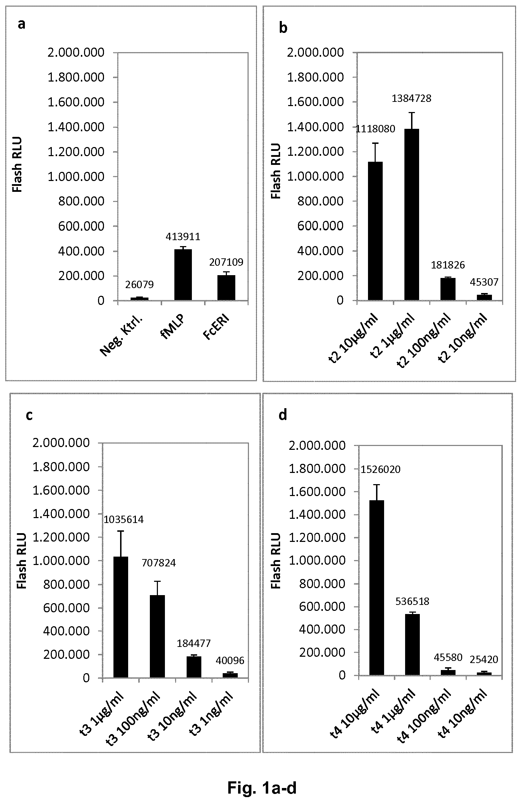

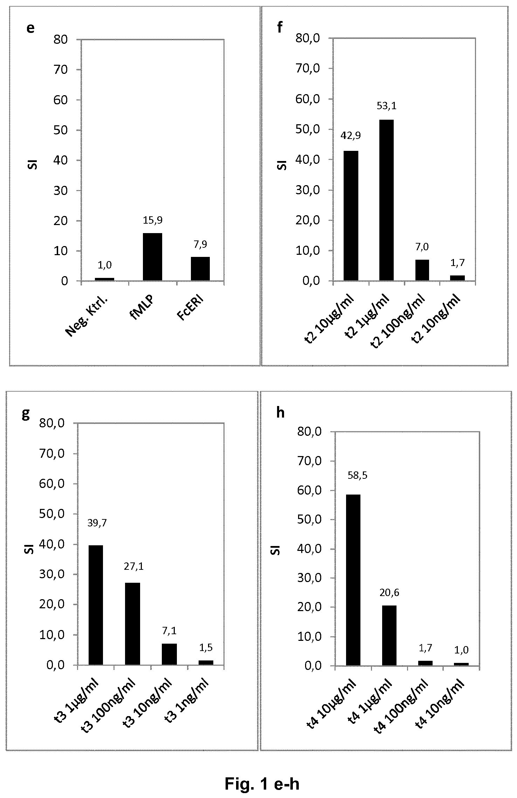

**Fig. 1** zeigt die Resultate des erfindungsgemäßen Verfahrens gemessen in Flash Chemilumineszenz relative light units (RLU; **Fig. 1a****-d).** Jeweils 50µl Vollblut eines Pollenallergikers wurden mit Allergenextrakten (t2: Erlenpollenextrakt; t3: Birkenpollenextrakt; t4: Haselpollenextrakt) in verschiedenen Konzentrationen (10µg - 1ng) bzw. Kontrollen (anti-FcεRl und fMLP) stimuliert und die Basophilenaktivierung mittels Chemilumineszenztest bestimmt. Die Balkendiagramme zeigen jeweils die absoluten RLU Meßwerte in Abhängigkeit von den Allergenkonzentrationen bzw. den Kontrollen. Die Messwerte sind zusätzlich über den Balken angegeben. **Fig. 1a** zeigt die Reaktion der nicht stimulierten Kontrolle (neg. Ktrl.), sowie der Kontrollstimulation mit anti-FcεRI und fMLP. **Fig. 1b** zeigt die Reaktion der mit unterschiedlichen Konzentrationen (10 µg/ml, 1 µg/ml, 100 ng/ml, 10 ng/ml) Erlenpollenextrakt stimulierten Proben. **Fig. 1c** zeigt die Reaktion der mit unterschiedlichen Konzentrationen (1 µg/ml, 100 ng/ml, 10 ng/ml, 1 ng/ml) Birkenpollenextrakt stimulierten Proben. **Fig. 1d** zeigt die Reaktion der mit unterschiedlichen Konzentrationen (10 µg/ml, 1 µg/ml, 100 ng/ml, 10 ng/ml) Haselpollenextrakt stimulierten Proben. Die Bestimmung erfolgte im Dreifachansatz. Es wurden Mittelwerte und Standardabweichungen berechnet. In **Fig. 1 e-h** ist der Stimulationsindex (SI) berechnet durch (Mittelwert RLU aktivierte Probe)/ (Mittelwert RLU neg. Kontrolle) für die in **Fig. 1 a-d** dargestellten Proben gezeigt.

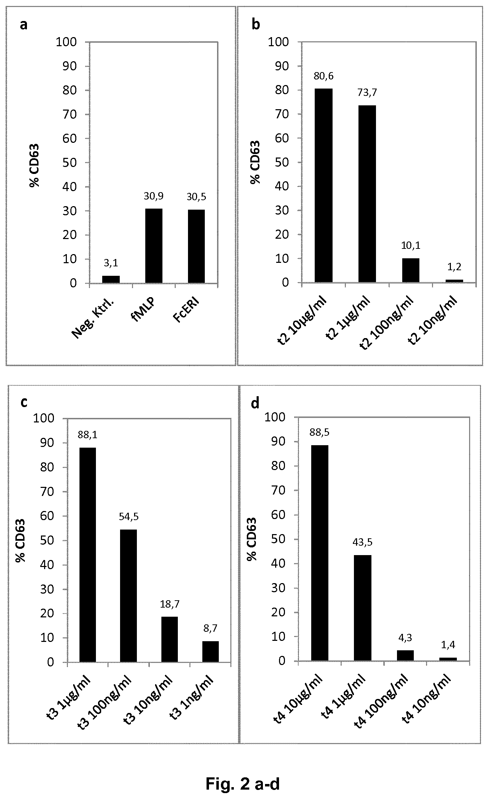

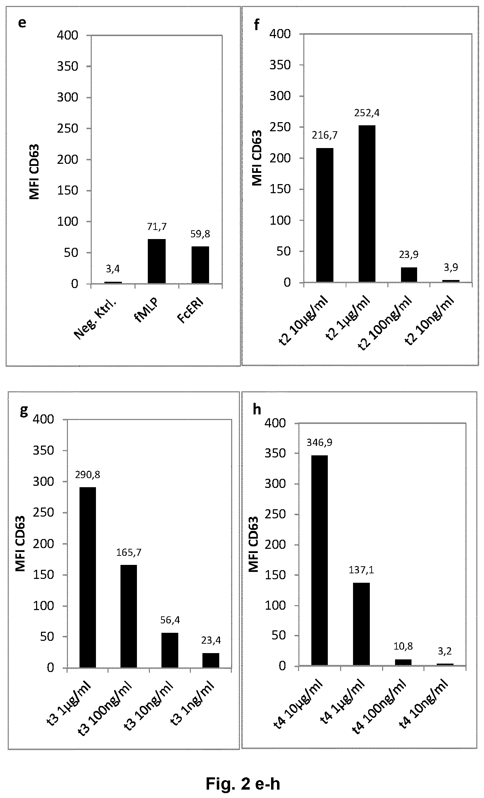

**Fig.** 2 zeigt einen Basophilenaktivierungstest mit durchflusszytometrischer Bestimmung der aktivierten Basophilen in % **(****Fig. 2a****-d)** oder als mittlere Fluoreszenzintensität (MFI; **Fig. 2e****-h).** Jeweils 50µl Vollblut eines Pollenallergikers wurden mit den gleichen Allergenextrakten wie im Versuch zu **Fig. 1** (t2: Erlenpollenextrakt; t3: Birkenpollenextrakt; t4: Haselpollenextrakt) in verschiedenen Konzentrationen (10µg - 1ng) bzw. Kontrollen (anti-FcεRI und fMLP) stimuliert und die Basophilenaktivierung mittels Durchflusszytometrie bestimmt. Die Balkendiagramme zeigen jeweils die Anteile aktivierter Basophiler in Abhängigkeit von den Allergenkonzentrationen bzw. den Kontrollen. Die Messwerte sind zusätzlich über den Balken angegeben. **Fig. 2a** zeigt die Reaktion der nicht stimulierten Kontrolle (Negativkontrolle), sowie der Kontrollstimulation mit anti-FcεRI und fMLP. Dabei wurden in beiden Positivkontrollen ca. 30% der Basophilen aktiviert. Ab einer Stimulation von mehr als 10% wurde die Reaktion als positiv bewertet. **Fig. 2b** zeigt die Reaktion der mit unterschiedlichen Konzentrationen (10 µg/ml, 1 µg/ml, 100 ng/ml, 10 ng/ml) Erlenpollenextrakt stimulierten Proben. **Fig. 2c** zeigt die Reaktion der mit unterschiedlichen Konzentrationen (1 µg/ml, 100 ng/ml, 10 ng/ml, 1 ng/ml) Birkenpollenextrakt stimulierten Proben. **Fig. 2d** zeigt die Reaktion der mit unterschiedlichen Konzentrationen (10 µg/ml, 1 µg/ml, 100 ng/ml, 10 ng/ml) Haselpollenextrakt stimulierten Proben. Zellen mit einem CD123+/HLA-DR- Phänotyp wurden als Basophile identifiziert, die Aktivierung wurde über anti-CD63-Vio-Blue detektiert.

Die maximale Reaktivität der Basophilen lag abhängig vom Pollenextrakt bei 80-88% bei der jeweils höchsten eingesetzten Konzentration und nahm mit zunehmender Verdünnung der Allergenextrakte stetig ab.

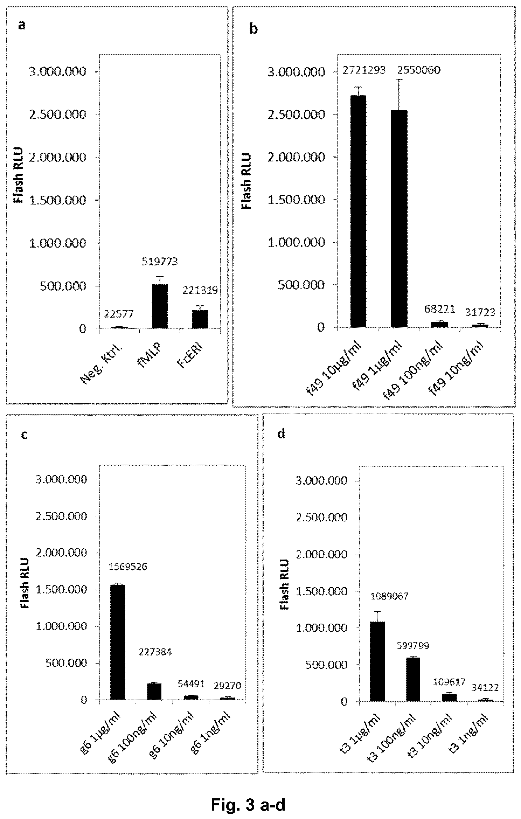

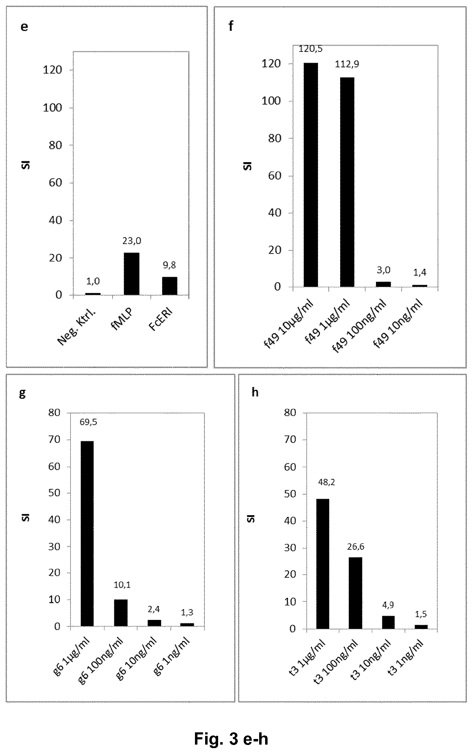

**Fig. 3** zeigt die Resultate des erfindungsgemäßen Verfahrens gemessen in Flash Chemilumineszenz relative light units (RLU; **Fig. 3a****-d).** Jeweils 50µl Vollblut eines Pollenallergikers wurden mit Allergenextrakten (f49: Apfelextrakt; g6: Lieschgraspollenextrakt; t3: Birkenpollenextrakt) in verschiedenen Konzentrationen (10µg - 1ng) bzw. Kontrollen (anti-FcεRl und fMLP) stimuliert und die Basophilenaktivierung mittels Chemilumineszenztest bestimmt. Die Balkendiagramme zeigen jeweils die absoluten RLU Meßwerte in Abhängigkeit von den Allergenkonzentrationen bzw. den Kontrollen. Die Messwerte sind zusätzlich über den Balken angegeben. **Fig. 3a** zeigt die Reaktion der nicht stimulierten Kontrolle (neg. Ktrl.), sowie der Kontrollstimulation mit anti-FcεRI und fMLP. **Fig. 3b** zeigt die Reaktion der mit unterschiedlichen Konzentrationen (10 µg/ml, 1 µg/ml, 100 ng/ml, 10 ng/ml) Apfelextrakt stimulierten Proben. **Fig. 3c** zeigt die Reaktion der mit unterschiedlichen Konzentrationen (1 µg/ml, 100 ng/ml, 10 ng/ml, 1 ng/ml) Lieschgraspollenextrakt stimulierten Proben. **Fig. 3d** zeigt die Reaktion der mit unterschiedlichen Konzentrationen (1 µg/ml, 100 ng/ml, 10 ng/ml, 1 ng/ml) Birkenpollenextrakt stimulierten Proben. Die Bestimmung erfolgte im Dreifachansatz. Es wurden Mittelwerte und Standardabweichungen berechnet. In **Fig. 3 e-h** ist der Stimulationsindex (SI) berechnet durch (Mittelwert RLU aktivierte Probe)/ (Mittelwert RLU neg. Kontrolle) für die in **Fig. 3 a-d** dargestellten Proben gezeigt.

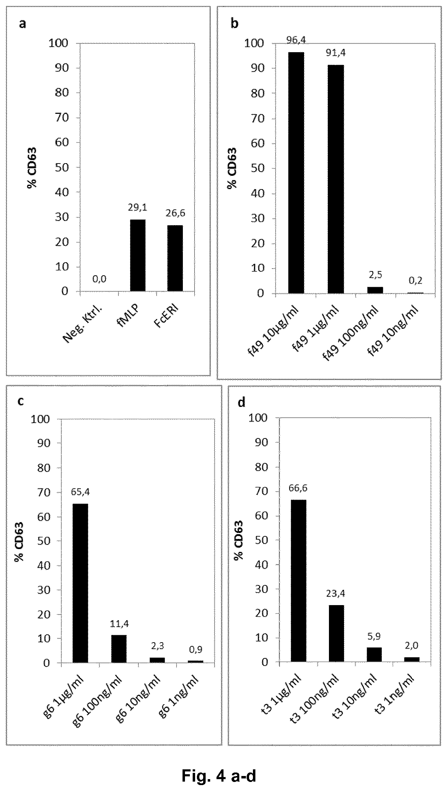

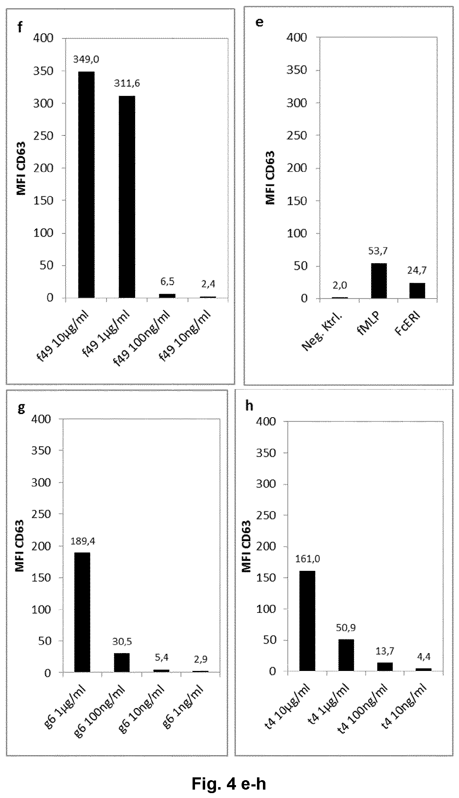

**Fig. 4** zeigt einen Basophilenaktivierungstest mit durchflusszytometrischer Bestimmung der aktivierten Basophilen in % **(****Fig. 4a****-d)** oder als mittlere Fluoreszenzintensität (MFI; **Fig. 4e****-h).** Jeweils 50µl Vollblut eines Pollenallergikers wurden mit Allergenextrakten (f49: Apfelextrakt; g6: Lieschgraspollenextrakt; t3: Birkenpollenextrakt) in verschiedenen Konzentrationen (10µg - 1ng) bzw. Kontrollen (anti-FcεRI und fMLP) stimuliert und die Basophilenaktivierung mittels Durchflusszytometrie bestimmt. Die Balkendiagramme zeigen jeweils die Anteile aktivierter Basophiler in Abhängigkeit von den Allergenkonzentrationen bzw. den Kontrollen. Die Messwerte sind zusätzlich über den Balken angegeben. **Fig. 4a** zeigt die Reaktion der nicht stimulierten Kontrolle (Negativkontrolle), sowie der Kontrollstimulation mit anti-FcεRI und fMLP. Dabei wurden in beiden Positivkontrollen ca. 30% der Basophilen aktiviert. Ab einer Stimulation von mehr als 10% wurde die Reaktion als positiv bewertet. **Fig. 4b** zeigt die Reaktion der mit unterschiedlichen Konzentrationen (10 µg/ml, 1 µg/ml, 100 ng/ml, 10 ng/ml) Apfelextrakt stimulierten Proben. **Fig. 4c** zeigt die Reaktion der mit unterschiedlichen Konzentrationen (1 µg/ml, 100 ng/ml, 10 ng/ml, 1 ng/ml) Lieschgraspollenextrakt stimulierten Proben. **Fig. 4d** zeigt die Reaktion der mit unterschiedlichen Konzentrationen (1 µg/ml, 100 ng/ml, 10 ng/ml, 1 ng/ml) Birkenpollenextrakt stimulierten Proben. Zellen mit einem CD123+/HLA-DR- Phänotyp wurden als Basophile identifiziert, die Aktivierung wurde über anti-CD63-Vio-Blue detektiert.

Die maximale Reaktivität der Basophilen lag abhängig vom Extrakt bei 96-65% bei der jeweils höchsten eingesetzten Konzentration und nahm mit zunehmender Verdünnung der Allergenextrakte stetig ab.

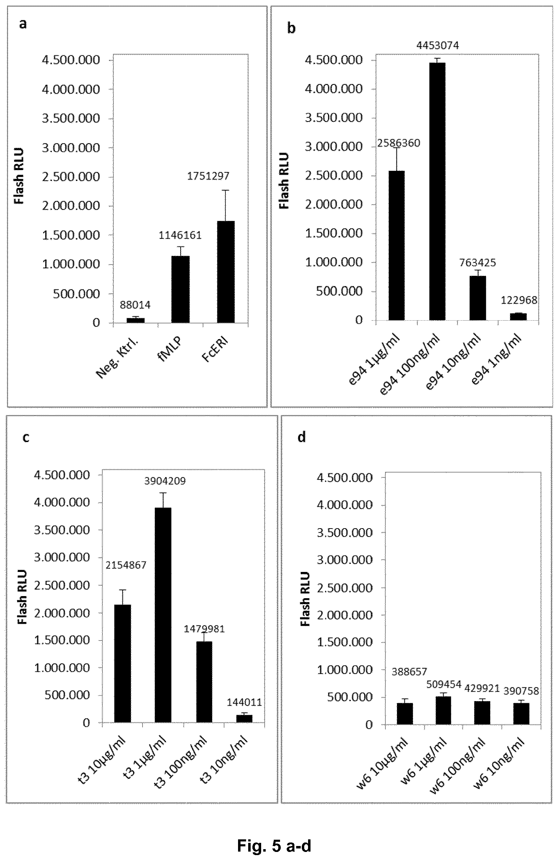

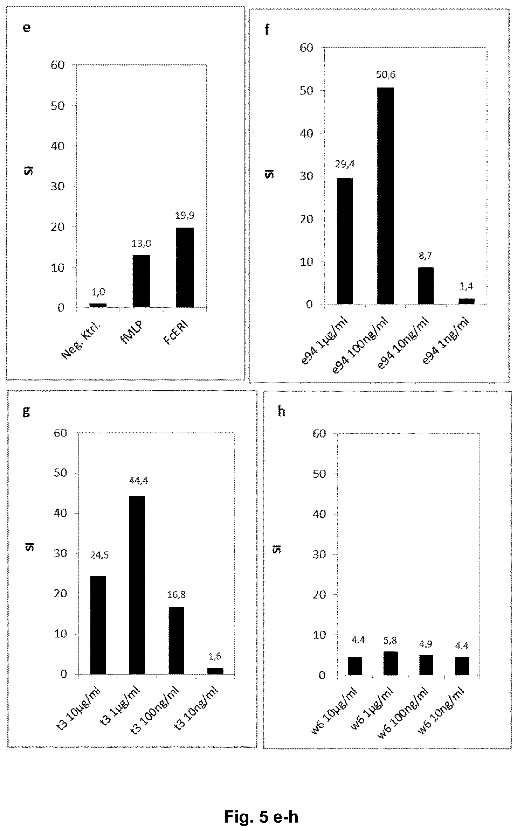

**Fig. 5** zeigt die Resultate des erfindungsgemäßen Verfahrens gemessen in Flash Chemilumineszenz relative light units (RLU; **Fig. 5a****-d).** Jeweils 50µl Vollblut eines Pollenallergikers wurden mit Allergenextrakten (e94: rFeld d 1; t3: Birkenpollenextrakt; w6: Beifußpollenextrakt) in verschiedenen Konzentrationen (10µg - 1ng) bzw. Kontrollen (anti-FcεRI und fMLP) stimuliert und die Basophilenaktivierung mittels Chemilumineszenztest bestimmt. Die Balkendiagramme zeigen jeweils die absoluten RLU Meßwerte in Abhängigkeit von den Allergenkonzentrationen bzw. den Kontrollen. Die Messwerte sind zusätzlich über den Balken angegeben. **Fig. 5a** zeigt die Reaktion der nicht stimulierten Kontrolle (neg. Ktrl.), sowie der Kontrollstimulation mit anti-FcεRI und fMLP. **Fig. 5b** zeigt die Reaktion der mit unterschiedlichen Konzentrationen (1 µg/ml, 100 ng/ml, 10 ng/ml, 1 ng/ml) rFel d 1 stimulierten Proben. **Fig. 5c** zeigt die Reaktion der mit unterschiedlichen Konzentrationen (10 µg/ml, 1 µg/ml, 100 ng/ml, 10 ng/ml) Birkenpollenextrakt stimulierten Proben. **Fig. 5d** zeigt die Reaktion der mit unterschiedlichen Konzentrationen (10 µg/ml, 1 µg/ml, 100 ng/ml, 10 ng/ml) Beifußpollenextrakt stimulierten Proben. Die Bestimmung erfolgte im Dreifachansatz. Es wurden Mittelwerte und Standardabweichungen berechnet. In **Fig. 5 e-h** ist der Stimulationsindex (SI) berechnet durch (Mittelwert RLU aktivierte Probe)/ (Mittelwert RLU neg. Kontrolle) für die in **Fig. 5 a-d** dargestellten Proben gezeigt.

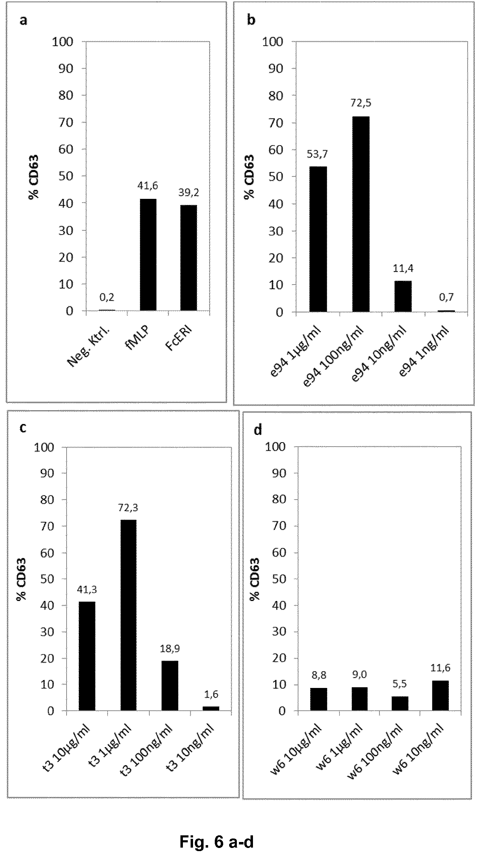

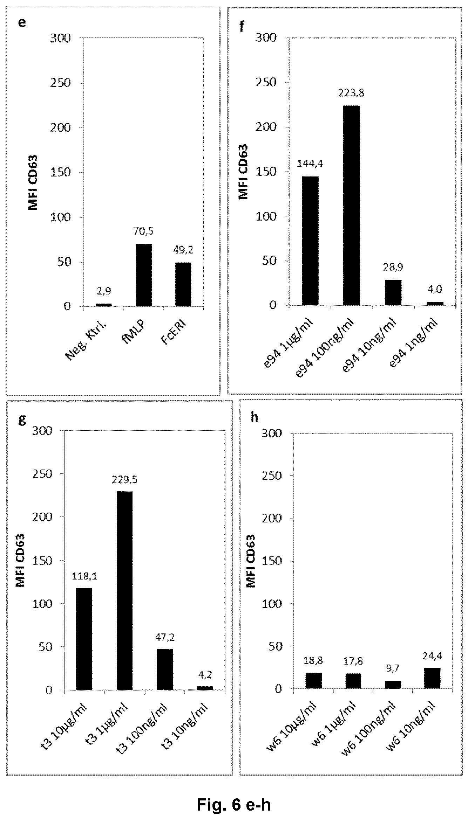

**Fig. 6** zeigt einen Basophilenaktivierungstest mit durchflusszytometrischer Bestimmung der aktivierten Basophilen in % (Fig. 6a-d) oder als mittlere Fluoreszenzintensität (MFI; **Fig. 6e****-h).** Jeweils 50µl Vollblut eines Pollenallergikers wurden mit Allergenextrakten (e94: rFeld d 1; t3: Birkenpollenextrakt; w6: Beifußpollenextrakt) in verschiedenen Konzentrationen (10µg - 1ng) bzw. Kontrollen (anti-FcεRI und fMLP) stimuliert und die Basophilenaktivierung mittels Durchflusszytometrie bestimmt. Die Balkendiagramme zeigen jeweils die Anteile aktivierter Basophiler in Abhängigkeit von den Allergenkonzentrationen bzw. den Kontrollen. Die Messwerte sind zusätzlich über den Balken angegeben. **Fig. 6a** zeigt die Reaktion der nicht stimulierten Kontrolle (Negativkontrolle), sowie der Kontrollstimulation mit anti-FcεRI und fMLP. Dabei wurden in beiden Positivkontrollen ca. 40% der Basophilen aktiviert. Ab einer Stimulation von mehr als 10% wurde die Reaktion als positiv bewertet. **Fig. 6b** zeigt die Reaktion der mit unterschiedlichen Konzentrationen (1 µg/ml, 100 ng/ml, 10 ng/ml, 1 ng/ml) rFel d 1 stimulierten Proben. **Fig. 6c** zeigt die Reaktion der mit unterschiedlichen Konzentrationen (10 µg/ml, 1 µg/ml, 100 ng/ml, 10 ng/ml) Birkenpollenextrakt stimulierten Proben. **Fig. 6d** zeigt die Reaktion der mit unterschiedlichen Konzentrationen (10 µg/ml, 1 µg/ml, 100 ng/ml, 10 ng/ml) Beifußpollenextrakt stimulierten Proben. Zellen mit einem CD123+/HLA-DR- Phänotyp wurden als Basophile identifiziert, die Aktivierung wurde über anti-CD63-Vio-Blue detektiert.

Die maximale Reaktivität der Basophilen lag abhängig vom Extrakt bei 73-12%.

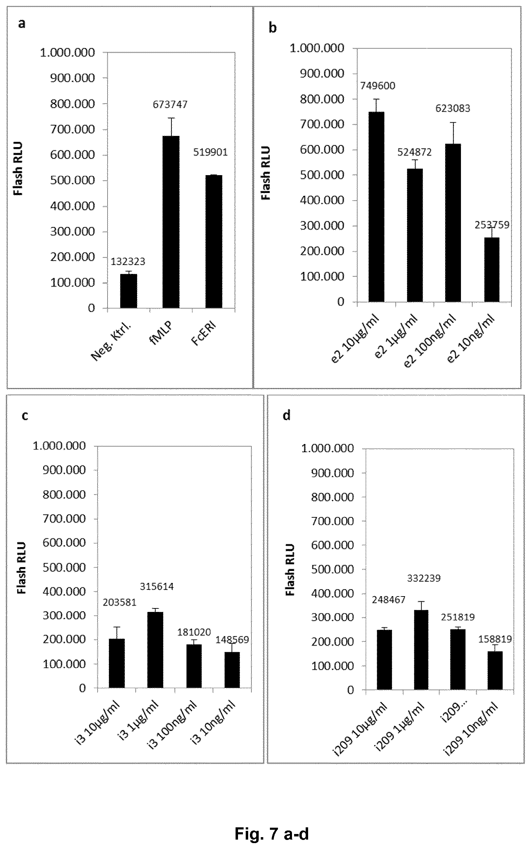

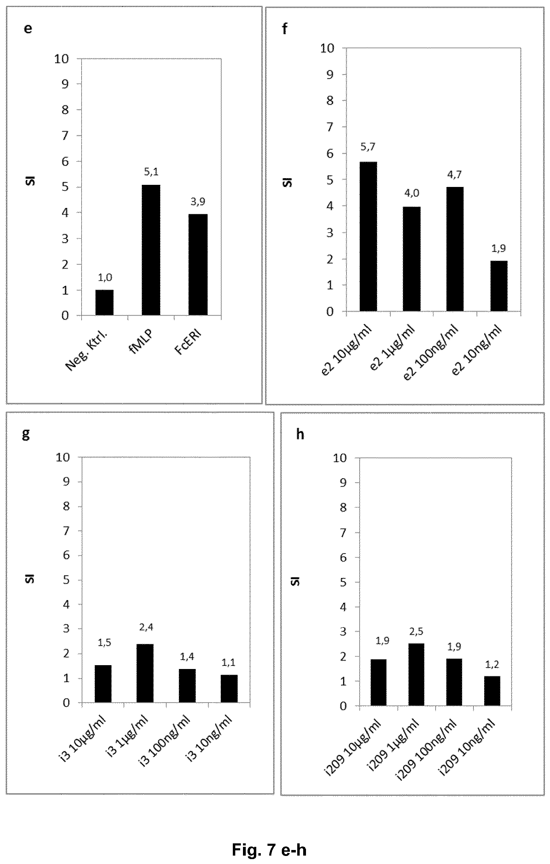

**Fig. 7** zeigt die Resultate des erfindungsgemäßen Verfahrens gemessen in Flash Chemilumineszenz relative light units (RLU; **Fig. 7a****-d).** Jeweils 50µl Vollblut eines Pollenallergikers wurden mit Allergenextrakten (e2: Hundehaarextrakt; i3: Wespengiftextrakt; i209: rVes v 5) in verschiedenen Konzentrationen (10µg - 1ng) bzw. Kontrollen (anti-FcεRI und fMLP) stimuliert und die Basophilenaktivierung mittels Chemilumineszenztest bestimmt. Die Balkendiagramme zeigen jeweils die absoluten RLU Meßwerte in Abhängigkeit von den Allergenkonzentrationen bzw. den Kontrollen. Die Messwerte sind zusätzlich über den Balken angegeben. **Fig. 7a** zeigt die Reaktion der nicht stimulierten Kontrolle (neg. Ktrl.), sowie der Kontrollstimulation mit anti-FcεRI und fMLP. **Fig. 7b** zeigt die Reaktion der mit unterschiedlichen Konzentrationen (10 µg/ml, 1 µg/ml, 100 ng/ml, 10 ng/ml) Hundehaarextrakt stimulierten Proben. **Fig. 7c** zeigt die Reaktion der mit unterschiedlichen Konzentrationen (10 µg/ml, 1 µg/ml, 100 ng/ml, 10 ng/ml) Wespengiftextrakt stimulierten Proben. **Fig. 7d** zeigt die Reaktion der mit unterschiedlichen Konzentrationen (10 µg/ml, 1 µg/ml, 100 ng/ml, 10 ng/ml) rVes v 5 stimulierten Proben. Die Bestimmung erfolgte im Dreifachansatz. Es wurden Mittelwerte und Standardabweichungen berechnet. In **Fig. 7 e-h** ist der Stimulationsindex (SI) berechnet durch (Mittelwert RLU aktivierte Probe)/ (Mittelwert RLU neg. Kontrolle) für die in **Fig. 7 a-d** dargestellten Proben gezeigt.

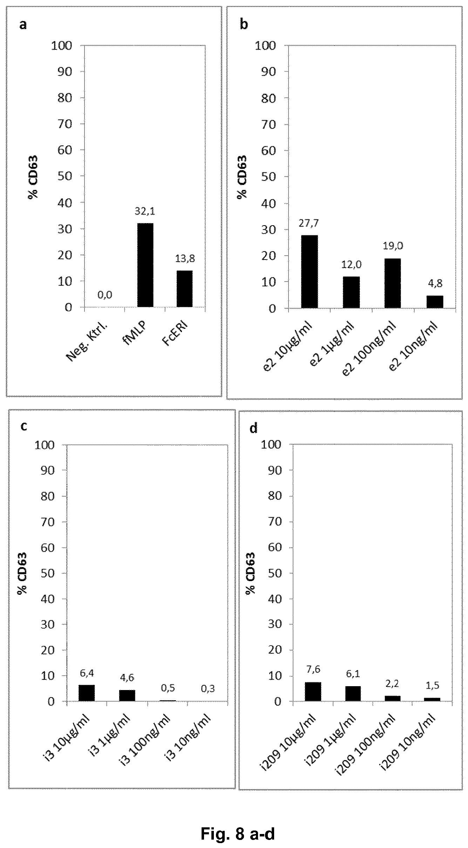

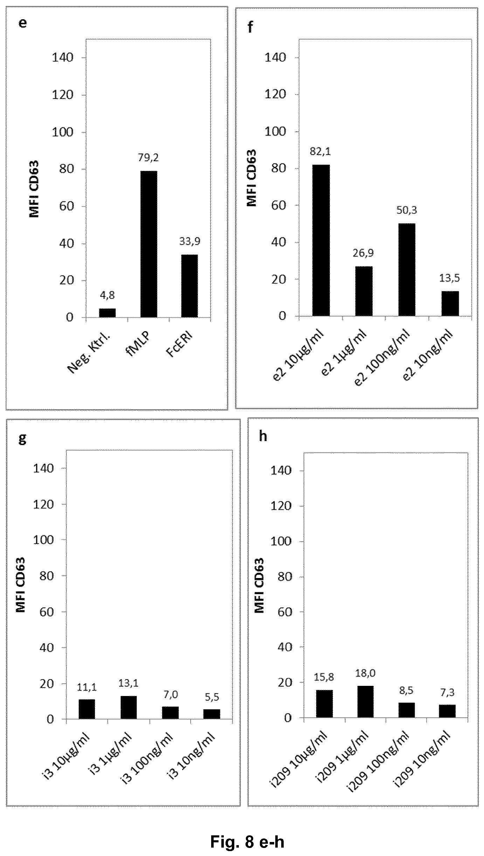

**Fig.** 8 zeigt einen Basophilenaktivierungstest mit durchflusszytometrischer Bestimmung der aktivierten Basophilen in % **(****Fig. 8a****-d)** oder als mittlere Fluoreszenzintensität (MFI; **Fig. 8e****-h).** Jeweils 50µl Vollblut eines Pollenallergikers wurden mit Allergenextrakten (e2: Hundehaarextrakt; i3: Wespengiftextrakt; i209: rVes v 5) in verschiedenen Konzentrationen (10µg - 1ng) bzw. Kontrollen (anti-FcεRI und fMLP) stimuliert und die Basophilenaktivierung mittels Durchflusszytometrie bestimmt. Die Balkendiagramme zeigen jeweils die Anteile aktivierter Basophiler in Abhängigkeit von den Allergenkonzentrationen bzw. den Kontrollen. Die Messwerte sind zusätzlich über den Balken angegeben. **Fig. 8a** zeigt die Reaktion der nicht stimulierten Kontrolle (Negativkontrolle), sowie der Kontrollstimulation mit anti-FcεRI und fMLP. Dabei wurden in beiden Positivkontrollen ca. 30% der Basophilen aktiviert. Ab einer Stimulation von mehr als 10% wurde die Reaktion als positiv bewertet. **Fig. 8b** zeigt die Reaktion der mit unterschiedlichen Konzentrationen (1 µg/ml, 100 ng/ml, 10 ng/ml, 1 ng/ml) Hundehaarextrakt stimulierten Proben. **Fig. 8c** zeigt die Reaktion der mit unterschiedlichen Konzentrationen (1 µg/ml, 100 ng/ml, 10 ng/ml, 1 ng/ml) Wespengiftextrakt stimulierten Proben. **Fig. 8d** zeigt die Reaktion der mit unterschiedlichen Konzentrationen (10 µg/ml, 1 µg/ml, 100 ng/ml, 10 ng/ml) rVes v 5 stimulierten Proben. Zellen mit einem CD123+/HLA-DR- Phänotyp wurden als Basophile identifiziert, die Aktivierung wurde über anti-CD63-Vio-Blue detektiert.

Die maximale Reaktivität der Basophilen lag abhängig vom Extrakt bei 25-8%.

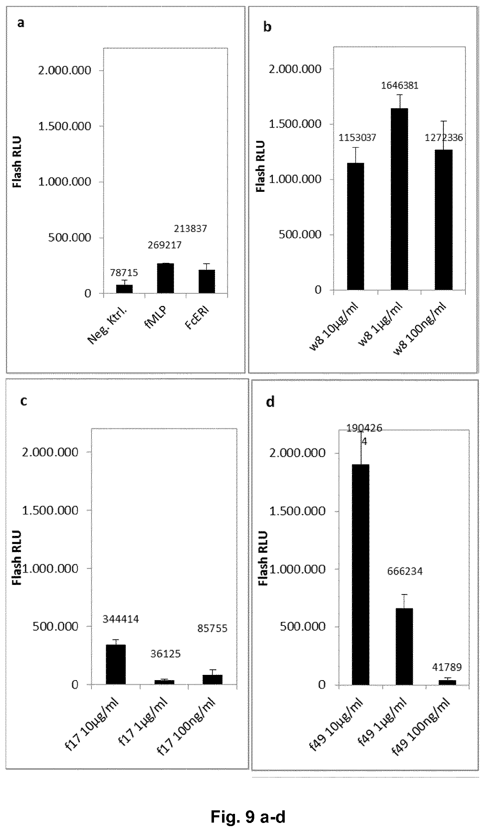

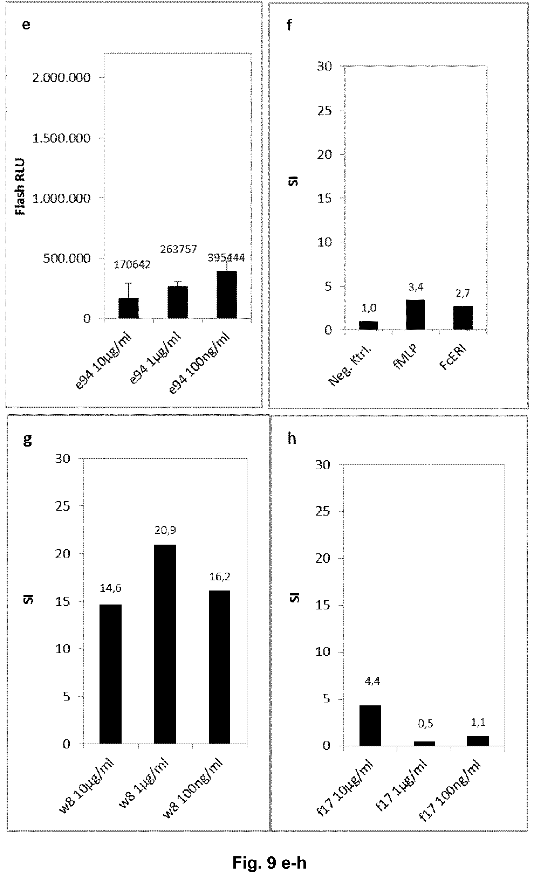

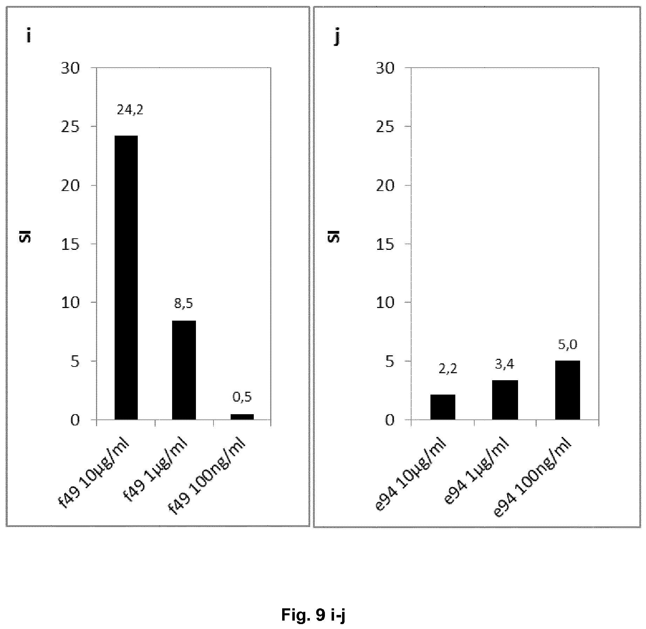

**Fig. 9** zeigt die Resultate des erfindungsgemäßen Verfahrens gemessen in Flash Chemilumineszenz relative light units (RLU; **Fig. 9a****-d).** Jeweils 50µl Vollblut eines Pollenallergikers wurden mit Allergenextrakten (w8: Löwenzahnpollenextrakt; f17: Haselnussextrakt; f49: Apfelextrakt; e94 rFel d 1) in verschiedenen Konzentrationen (10µg - 100ng) bzw. Kontrollen (anti-FcεRI und fMLP) stimuliert und die Basophilenaktivierung mittels Chemilumineszenztest bestimmt. Die Balkendiagramme zeigen jeweils die absoluten RLU Meßwerte in Abhängigkeit von den Allergenkonzentrationen bzw. den Kontrollen. Die Messwerte sind zusätzlich über den Balken angegeben. **Fig. 9a** zeigt die Reaktion der nicht stimulierten Kontrolle (neg. Ktrl.), sowie der Kontrollstimulation mit anti-FcεRI und fMLP. **Fig. 9b** zeigt die Reaktion der mit unterschiedlichen Konzentrationen (10 µg/ml, 1 µg/ml, 100 ng/ml) Löwenzahnpollenextrakt stimulierten Proben. **Fig. 9c** zeigt die Reaktion der mit unterschiedlichen Konzentrationen (10 µg/ml, 1 µg/ml, 100 ng/ml) Haselnussextrakt stimulierten Proben. **Fig. 9d** zeigt die Reaktion der mit unterschiedlichen Konzentrationen (10 µg/ml, 1 µg/ml, 100 ng/ml) Apfelextrakt stimulierten Proben. **Fig. 9e** zeigt die Reaktion der mit unterschiedlichen Konzentrationen (10 µg/ml, 1 µg/ml, 100 ng/ml) rFel d 1 stimulierten Proben. Die Bestimmung erfolgte im Dreifachansatz. Es wurden Mittelwerte und Standardabweichungen berechnet. In **Fig. 9 f-j** ist der Stimulationsindex (SI) berechnet durch (Mittelwert RLU aktivierte Probe)/ (Mittelwert RLU neg. Kontrolle) für die in **Fig. 9 a-e** dargestellten Proben gezeigt.

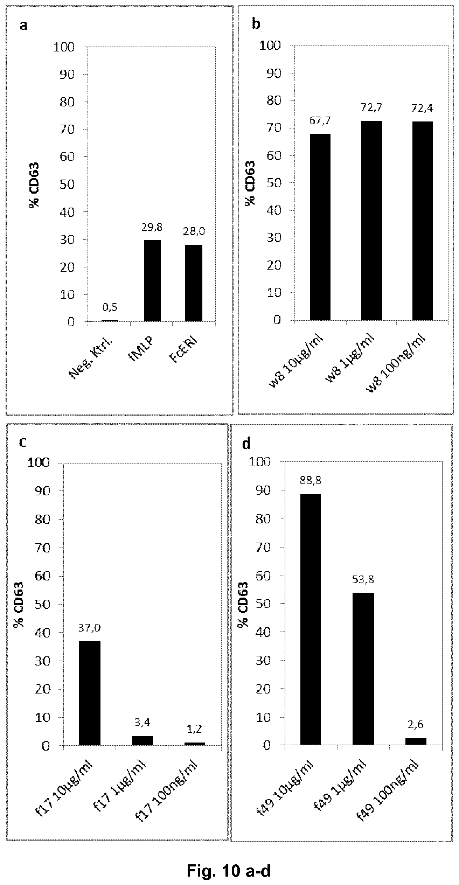

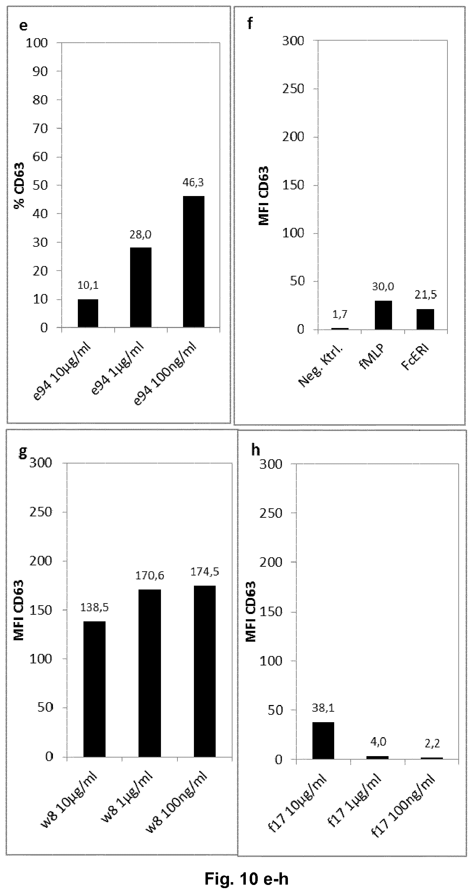

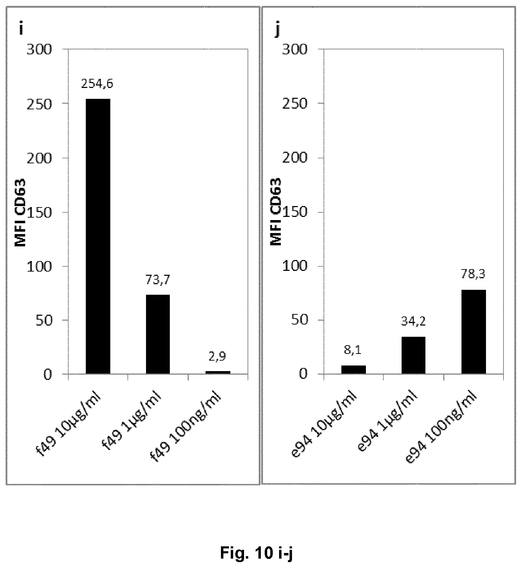

**Fig. 10** zeigt einen Basophilenaktivierungstest mit durchflusszytometrischer Bestimmung der aktivierten Basophilen in % **(****Fig. 10a****-e)** oder als mittlere Fluoreszenzintensität (MFI; **Fig. 10f****-j).** Jeweils 50µl Vollblut eines Pollenallergikers wurden mit Allergenextrakten (w8: Löwenzahnpollenextrakt; f17: Haselnussextrakt; f49: Apfelextrakt; e94 rFel d 1) in verschiedenen Konzentrationen (10µg - 100ng) bzw. Kontrollen (anti-FcεRI und fMLP) stimuliert und die Basophilenaktivierung mittels Durchflusszytometrie bestimmt. Die Balkendiagramme zeigen jeweils die Anteile aktivierter Basophiler in Abhängigkeit von den Allergenkonzentrationen bzw. den Kontrollen. Die Messwerte sind zusätzlich über den Balken angegeben. **Fig. 10a** zeigt die Reaktion der nicht stimulierten Kontrolle (Negativkontrolle), sowie der Kontrollstimulation mit anti-FcεRI und fMLP. Dabei wurden in beiden Positivkontrollen ca. 30% der Basophilen aktiviert. Ab einer Stimulation von mehr als 10% wurde die Reaktion als positiv bewertet. **Fig. 10b** zeigt die Reaktion der mit unterschiedlichen Konzentrationen (10 µg/ml, 1 µg/ml, 100 ng/ml) Löwenzahnpollenextrakt stimulierten Proben. **Fig. 10c** zeigt die Reaktion der mit unterschiedlichen Konzentrationen (10 µg/ml, 1 µg/ml, 100 ng/ml) Haselnussextrakt stimulierten Proben. **Fig. 10d** zeigt die Reaktion der mit unterschiedlichen Konzentrationen (10 µg/ml, 1 µg/ml, 100 ng/ml) Apfelextrakt stimulierten Proben. **Fig. 10e** zeigt die Reaktion der mit unterschiedlichen Konzentrationen (10 µg/ml, 1 µg/ml, 100 ng/ml) rFel d 1 stimulierten Proben. Zellen mit einem CD123+/HLA-DR- Phänotyp wurden als Basophile identifiziert, die Aktivierung wurde über anti-CD63-Vio-Blue detektiert.

Die maximale Reaktivität der Basophilen lag abhängig vom Extrakt bei 89-37%.

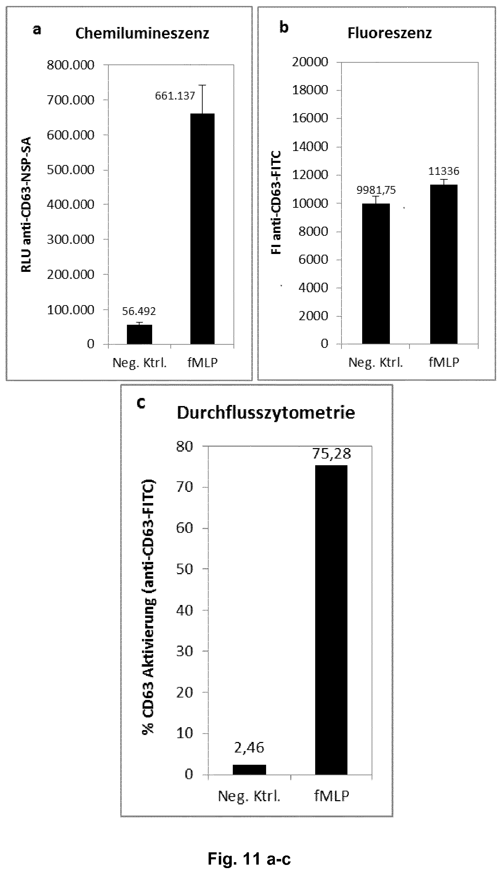

**Fig. 11** zeigt die Resultate einer Patientenprobe nach Stimulation mit fMLP bzw. der nicht stimulierten Kontrolle (neg. Ktrl.) mit unterschiedlichen Detektionssystemen. **Fig. 11a** zeigt die Resultate des erfindungsgemäßen Verfahrens gemessen in Flash Chemilumineszenz relative light units (RLU). **Fig. 11b** zeigt die Resultate der nach dem erfindungsgemäßen Verfahren durchgeführten Anreicherung der Basophilen, nach Detektion mit einem Fluoreszenz-markierten Antikörper (anti-CD63-FITC; 2µg/ml) gemessen als Fluoreszenzintensität (FI). **Fig. 11c** zeigt einen Basophilenaktivierungstest mit durchflusszytometrischer Bestimmung der aktivierten Basophilen. Jeweils 50µl Vollblut eines Pollenallergikers wurden mit fMLP stimuliert bzw. ohne Stimulation (neg. Ktrl.) belassen. Die Basophilenaktivierung wurde dann mit den oben angegebenen Methoden bestimmt. Die Balkendiagramme zeigen jeweils die absoluten Messwerte in RLU **(****Fig. 11a****)** oder FI **(****Fig. 11b****)** bzw. die Basophilenaktivierung in % **(****Fig. 11c****).**

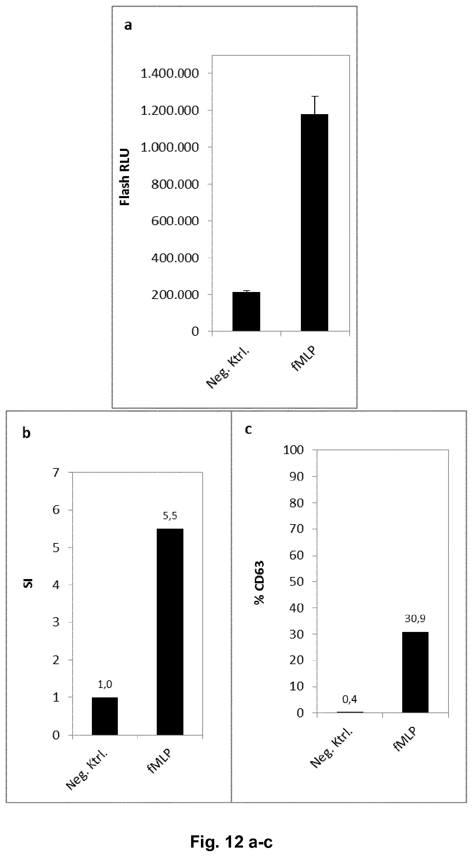

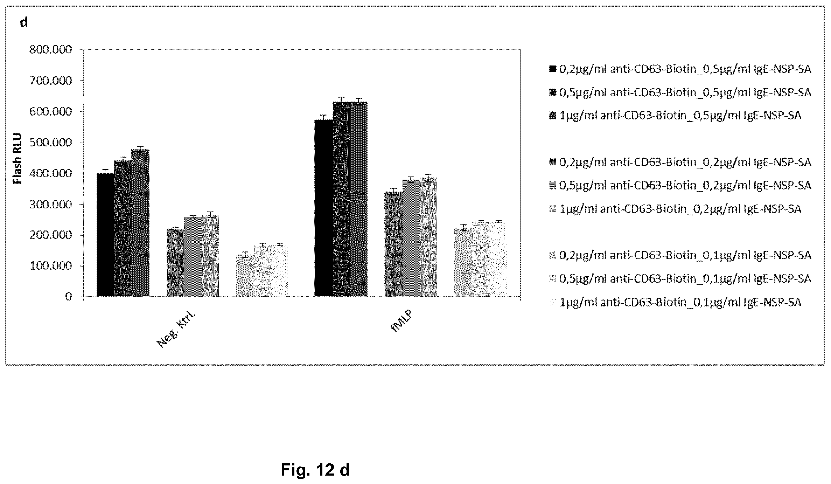

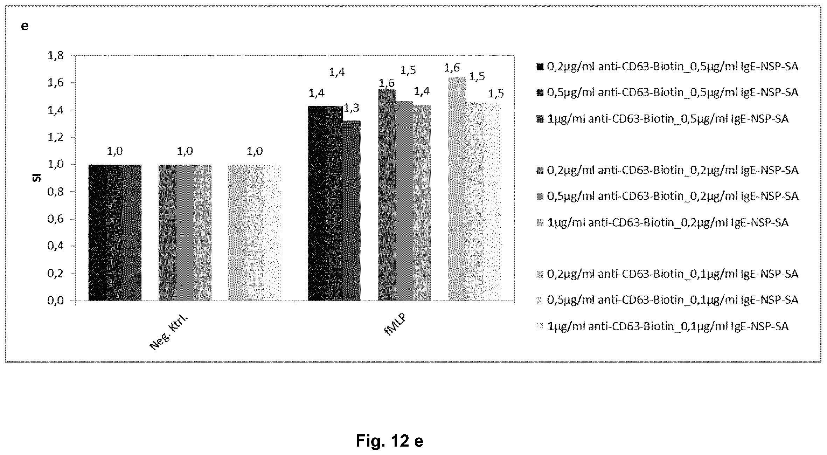

**Fig. 12** zeigt die Resultate einer Patientenprobe nach Stimulation mit fMLP bzw. der nicht stimulierten Kontrolle (neg. Ktrl.) unter Verwendung verschiedener Antikörperkombinationen für Anreicherung und Nachweis der Basophilen. Es wurde kein Allergen eingesetzt. **Fig. 12a** zeigt die Resultate des erfindungsgemäßen Verfahrens gemessen in Flash Chemilumineszenz relative light units (RLU). **Fig. 12b** zeigt den Stimulationsindex (SI)berechnet durch (Mittelwert RLU aktivierte Probe)/ (Mittelwert RLU neg. Kontrolle) für die in **Fig. 12a** dargestellte Probe. **Fig. 12c** zeigt einen Basophilenaktivierungstest mit durchflusszytometrischer Bestimmung der aktivierten Basophilen. **Fig. 12d** zeigt die Resultate einer Anreicherung der Basophilen über den aktivierungsabhängigen Marker CD63 über anti-CD63-Biotin und den Nachweis mit anti-IgE-NSP-SA. Der anti-CD63-Biotin Antikörper wurde in Konzentrationen von 0,2-1 µg/ml eingesetzt, während der Nachweisantikörper anti-lgE-NSP-SA in einer fixen Konzentration von 0,5 µg/ml eingesetzt wurde. **Fig. 12e** zeigt den Stimulationsindex (SI)berechnet durch (Mittelwert RLU aktivierte Probe)/ (Mittelwert RLU neg. Kontrolle) für die in **Fig. 12d** gezeigten Proben.

### Beispiele:

### 1a. Vorbereitung Allergenverdünnungen:

Zum Nachweis der Basophilenaktivierung wurde ein anti-CD63 Antikörper (Klon 7G3, BD Bioscience #353014) mit einem NSP-SA-NHS Acridinium Ester (Heliosense Biotechnologies, #199293-83-9) nach Herstellerangaben kovalent gekoppelt. Anschließend wurde der Antikörper über eine PD-10 Säule (GE Healthcare, #383420) gereinigt. Auf die gleiche Weise wurde IgE von Biomol GmbH mit NSP-SA-NHS markiert.

### 1b. Vorbereitung Allergenverdünnungen:

T2 Erlenpollenextrakt (Squarix, #T-4402), t3 Birkenpollenextrakt (Squarix, #T-4400) und t4 Haselpollenextrakt (Squarix, #T-4404) wurden in Konzentrationen von 10 µg/ml, 1 µg/ml, 100 ng/ml, 10 ng/ml, 1 ng/ml in Verdünnungspuffer (1×Hanks Balanced Salt Solution (Thermo Fisher Scientific #14065056), 40mM Hepes, pH 7,4) verdünnt. Als Negativ-Kontrolle diente Verdünnungspuffer. Als Positivkontrollen wurde fMLP (Sigma, #F3506-5MG) in einer Konzentration von 10⁻⁶ M und anti-FcεRI Antikörper (Klon AER-37, ebioscience, #14-5899-82) in einer Konzentration von 100 ng/ml eingesetzt. Zudem wurden als Antigene Apfelextrakt (Squarix # F-2327), Lieschgraspollenextrakt (Squarix,# G-5501), e94 (aufgereinigt gemäß Rogers et al. (1993) Recombinant Fel d.I: Expression, purification, IgE binding and reaction with cat-allergic human T cells., Molecular Immunology, 30 April 2010 (6), 559-568), Beifußpollenextrakt (Squarix #W-3400), Hundehaarextrakt (Squarix # E-6617), Wespengiftextrakt (Squarix # I-7804), i209 (aufgereinigt gemäß Seismann et al. 2010, Clinical and Molecular Allergy, 8:7), Löwenzahnpollenextrakt (Squarix # W-3404) und Haselnussextrakt /Squarix, #T-4404) eingesetzt.

### 1c. Stimulation:

Je 50µl peripheres Vollblut (heparinisiert) wurde in einer Mikrotiterplatte mit je 50 µl Allergen- bzw. Kontrollverdünnung gemischt und 20 min bei 37 °C in einem Wärmeschrank inkubiert. Die Reaktion wurde durch Zugabe von 10 µl einer konzentrierten EDTA-Lösung (100 mM EDTA in PBS) gestoppt.

### 2a1. Antikörperinkubation:

Anschließend wurden zwei Antikörper zu den Proben gegeben:
1) anti-CD123-Biotin (Klon H5C6, Biolegend #353014) in einer finalen Konzentration von 0,5 µg/ml
2) anti-CD63-NSP-SA (Klon 7G3, BD Bioscience #353014) in einer finalen Konzentration von 2 µg/ml
   Die Proben wurden gemischt und 10 min bei Raumtemperatur inkubiert.

Alternativ wurden anti-CD63-Biotin (Biolegend 353017) und anti-CD63-FITC (Miltenyi 130-100-160) eingesetzt.

### 2a2. Erythrolyse:

Durch Zugabe von 100 µl eines Saponin-haltigen Lysepuffers (0,14% Saponin in PBS) und 10 min Inkubation bei RT wurden Erythrozyten lysiert. Anschließend wurde die Mikrotiterplatte 5 min bei 500 g zentrifugiert und der Überstand abgenommen.

### 2a3. Beadinkubation:

Die Proben wurden mit je 10µg Streptavidin-beschichteten Magnetbeads (Sera-Mag Speedbeads Streptavidin blocked, GE-Healthcare, #21152104010350) gemischt und für 10min bei RT inkubiert.

### 2a4. Waschen:

Die an die Beads gebundenen Zellen wurden 5x mit 300µl Waschpuffer (0,05% Tween, 2mM EDTA, 150mM NaCl, 0,05% NaN3) am Plattenwasher, z.B. Tecan Hydroflex ausgestattet mit einem MBS96 Magneten, gewaschen.

### 2a5. Detektion:

Bei der anschließenden Detektion wurde der NSP-SA Acridinium-Ester am CD63 Antikörper durch zwei Lösungen getriggert (H2O2; 0,1 M HNO3 und 0,25M NaOH (Chemiluminescence Detection Reagent Kit, Invitron Ltd., #IV1-001)). Das erzeugte Chemilumineszenz Signal (Flash-Lumineszenz) wurde am Plattenluminometer z.B. Berthold Centro LB 960 bei einer Wellenlänge von 425nm detektiert.

### 2a6. Auswertung:

Die Stärke der Aktivierung der Basophilen wurde über die Zunahme des Chemilumineszenz-Signals im Vergleich zur unstimulierten Kontrolle berechnet. Es kann ein Stimulationsindex (SI) aus relativen light units (RLU) der aktivierten Probe/ RLU der unstimulierten Probe berechnet werden. Der zu überschreitende Schwellenwert ist dabei von der Testsubstanz abhängig.

### 2b. Durchflusszytometrische Messung:

Für die durchflusszytometrische Bestimmung wurden die Proben nach der Aktivierung mit anti-CD123-PE (Miltenyi Biotec, #130-098-894), anti-HLA-DR-PerCPVio700 (Miltenyi Biotec, #130-103-873) und anti-CD63-Vio-Blue (Miltenyi Biotec, #130-100-154) Antikörpern nach Herstellerangaben für 15 min bei 4 °C lichtgeschützt inkubiert.

Nach Zugabe von 1ml 1x FACS-Lysing-Solution (BD Bioscience, #349202) und einer Inkubation für 10 min bei RT wurden die Proben bei 500 g, 5min zentrifugiert, der Überstand abgenommen und die Proben erneut mit je 500 µl Waschpuffer (PBS ohne Ca²⁺/Mg²⁺, 0,5% BSA, 2 mM EDTA, 0,05 % NaN3) gewaschen. Die Messung erfolgte am Durchflusszytometer z.B. MACS Quant (Miltenyi Biotec) innerhalb von 2 h. Zellen mit einem CD123⁺/HLA-DR⁻ Phänotyp wurden als Basophile identifiziert. Basophile waren aktiviert, wenn das CD63 Signal einen gewählten Schwellenwert überstieg. Eine Gesamtaktivierung von ≥ 10% wurde als positiv gewertet.

### 2c. Fluoreszenzmessung

Die Fluoreszenz von anti-CD63-FITC wurde mit einem BMG Labtech Fluostar (485nm/520nm)-Gerät gemessen.

### Ergebnisse:

Der Chemilumineszenztest **(****Fig. 1****)** wurde dann als positiv gewertet wenn das Signal der stimulierten Probe sich im Verhältnis zur Kontrolle verdoppelte (SI ≥ 2). Bei der Auswertung der durchflusszytometrischen Bestimmungen **(****Fig. 2****)** werden alle Proben als positiv gewertet, die eine Aktivierung von ≥ 10% zeigen. Der Vergleich des Chemilumineszenztests mit dem Durchflusszytometrietests zeigt eine gute Korrelation der Ergebnisse der beiden Testsysteme sowie eine sehr gute Reproduzierbarkeit. Der dynamische Messbereich des Chemilumineszenzsignals ist dabei deutlich breiter als beim Fluoreszenzsignal.

Bei der durchflusszytometrischen Auswertung müssen zur Definition der aktivierten und nichtaktivierten Zellpopulationen Analysefenster gesetzt werden (Gating), die für jeden Patienten individuell angepasst werden müssen und subjektiv vom jeweiligen Auswertenden gesetzt werden. Diese Möglichkeiten der varianten Auswertung ist beim Chemilumineszenztest nicht gegeben, da hier nur das Verhältnis von stimulierter Probe zur Kontrolle gebildet wird. Dies ermöglicht eine standardisierte Auswertung, die sich so bei einem durchflusszytometrischen Test nicht erreichen lässt. Aufgrund der Probenvorbereitung im Mikrotiterplatten-Format, der schnellen Messung und einer einfachen Auswertung wird zudem ein deutlich höherer Probendurchsatz ermöglicht.

Dieser Versuch zeigte zudem, dass der Einsatz Chemilumineszenz als Detektionssystem einen entscheidenden Vorteil darstellt: die Basophilen und weitere Zellen zeigen nur eine sehr geringe *Autochemilumineszenz.* Eine absolute Reinheit der Zielzellen bei der Messung ist daher nicht erforderlich.

Im Gegensatz besitzen insbesondere tote Zellen und Granulozyten eine hohe *Autofluoreszenz,* was eine Überlagerung des gewünschten Signals bei der Durchflusszytometrie verursachen würde und daher ein entsprechendes Gating voraussetzt. Bei Verwendung des CD123-Biotin Antikörpers zur Reinigung der Basophilen ist ein geringer Anteil an insbesondere dendritischen Zellen, die ebenfalls CD123 an der Oberfläche exprimieren, in den Proben enthalten. Diese exprimieren allerdings kein CD63, so dass das detektierte anti-CD63 Chemilumineszenzsignal ausschließlich auf Basophile zurückzuführen ist. Die Messung selbst wird durch das Vorhandensein von anderen nicht-markierten Zellen nicht beeinträchtigt.

Die Kombination der Chemilumineszenz als Nachweismethode mit der Anwesenheit von unfraktioniertem Vollblut führt daher zu einem hochempfindlichen Nachweisverfahren, mit dem eine Vielzahl von Proben bei maximaler Reproduzierbarkeit und Standardisierbarkeit parallel abgearbeitet werden kann.

In **Figs. 3-10** wird mit einer breiten Auswahl von Antigenen und Probenspendern gezeigt, dass das erfindungsgemäße Verfahren weit anwendbar ist. Dabei jeweils die erste Figur (z.B. **Fig. 3****)** das gleiche Experiment mit Chemilumineszenz und die darauf folgende **(****Fig. 4****)** mit Durchflusszytometrie, um die Vergleichbarkeit zu demonstrieren. Das Ergebnis ist, dass diese überraschend hoch ist. Das erfindungsgemäße Verfahren kann die Durchflusszytometrie daher ersetzen.

In **Fig. 11** werden die drei Nachweismethoden Chemilumineszenz, Fluoreszenz und Durchflusszytometrie direkt verglichen. Dabei wird deutlich, dass Chemilumineszenz der Durchflusszytometrie an Sensitivität ungefähr entspricht und deutlich besser als Fluoreszenz geeignet ist, zur Bestätigung, dass das erfindungsgemäße Verfahren die Durchflusszytometrie ersetzen kann.

Das in **Fig. 12** gezeigte Experiment demonstriert schließlich, dass das erfindungsgemäße Verfahren auch dann funktioniert, wenn man zur Anreicherung einen aktivierungsabhängigen und zur Detektion einen aktivierungsunabhängigen Liganden einsetzt, statt umgekehrt wie in den anderen Experimenten. Zwar ist der der Hintergrund hier bei der Chemilumineszenz im Vergleich zum Signal höher, aber das Signal vom Hintergrund noch deutlich zu unterscheiden.

## Patentansprüche

1. Verfahren zur Diagnose einer Allergie, umfassend die Schritte:
a) Kontaktieren von Basophilen aus einer Vollblutprobe eines Patienten mit einem Allergen in einer wässrigen Lösung in Anwesenheit des Vollblutes mit einem Volumenanteil des Vollblutes an der wässrigen Lösung von wenigstens 20 Volumenprozent unter Bedingungen, die eine Aktivierung der Basophilen durch das Allergen zulassen,
b) Anreichern der Basophilen aus a) und
c) Nachweisen aktivierter Basophiler,
wobei das Nachweisen in Schritt c) dadurch erreicht wird, dass ein an der Oberfläche von Basophilen lokalisierter Marker für Basophile mittels Chemilumineszenz detektiert wird,
wobei in Schritt b) Basophile dadurch angereichert werden, dass sie an einen Liganden binden, der spezifisch an ein Polypeptid bindet, das ein für aktivierte Basophile charakteristischer Marker ist,
und/oder das Nachweisen in Schritt c) dadurch erreicht wird, dass ein für aktivierte Basophile charakteristischer Marker mittels Chemilumineszenz detektiert wird.

2. Verfahren nach Anspruch 1, wobei es sich bei dem Vollblut um unprozessiertes Vollblut handelt.

3. Verfahren nach einem der Ansprüche 1 bis 2, wobei Schritt a) in einer wässrigen Lösung mit einem Vollblutanteil von wenigstens 30, bevorzugt 35, noch bevorzugter 40, noch bevorzugter 45 Volumenprozent durchgeführt wird.

4. Verfahren nach einem der Ansprüche 1 bis 3, umfassend den Schritt Stoppen der Aktivierung der Basophilen, bevorzugt vor Schritt b).

5. Verfahren nach einem der Ansprüche 1 bis 4, wobei in Schritt b) die Basophilen dadurch angereichert werden, dass sie an einen Liganden binden, der spezifisch an ein Polypeptid bindet, das bei Basophilen an der Zelloberfläche lokalisiert ist, bevorzugt unabhängig von ihrem Aktivierungszustand.

6. Verfahren nach Anspruch 5, wobei der Ligand ein monoklonaler Antikörper gegen das Polypeptid ist.

7. Verfahren nach einem der Ansprüche 1 bis 6, wobei in Schritt b) die Basophilen immobilisiert werden.

8. Verfahren nach Anspruch 7, wobei die Basophilen an einem Bead, bevorzugt einem magnetischen Bead, immobilisiert werden.

9. Verfahren nach einem der Ansprüche 7 bis 8, wobei die Basophilen nach dem Immobilisieren gewaschen werden.

10. Verfahren nach einem der Ansprüche 1 bis 9, wobei in Schritt b) Erythrozyten lysiert und entfernt werden.

11. Verfahren nach Anspruch 10, wobei die Erythrozyten vor dem Immobilisieren der Basophilen durch Zentrifugieren entfernt werden.

12. Verfahren nach Anspruch 1 bis 11, wobei das Nachweisen in Schritt c) mit Hilfe eines mit einer chemilumineszenzfähigen Markierung versehenen Liganden gegen den an der Oberfläche von Basophilen lokalisierten Marker durchgeführt wird, bevorzugt einen Marker, der für aktivierte Basophile charakteristisch ist.

13. Verfahren nach Anspruch 12, wobei es sich um den mit einer chemilumineszenzfähigen Markierung versehenen Liganden um einen monoklonalen Antikörper handelt, der selbst eine chemilumineszenzfähige Markierung aufweist oder an den ein sekundärer Antikörper mit einer chemilumineszenzfähigen Markierung bindet.

14. Verfahren nach Anspruch 1 bis 13, wobei die Schritte a), b) und c) in einem Well einer Mikrotiterplatte ausgeführt werden.

15. Verfahren nach Anspruch 1 bis 14, wobei Schritt a) vor den Schritten b) und c) durchgeführt wird.

16. Kit umfassend
ein Gefäß zur Kontaktierung der Basophilen mit dem Allergen, wobei es sich bei dem Gefäß bevorzugt um eine Mikrotiterplatte handelt,
optional ein Allergen,
einen Liganden, der spezifisch gegen ein an der Zelloberfläche von Basophilen lokalisiertes Polypeptid bindet, zur Anreicherung von Basophilen und einen Liganden gegen einen an der Oberfläche von Basophilen lokalisierten Marker zur Detektion,
wobei der Ligand, der spezifisch gegen den an der Oberfläche von Basophilen lokalisierten Marker bindet, zur Detektion eine chemilumineszenzfähige Markierung aufweist,
wobei der Ligand, der spezifisch gegen ein an der Zelloberfläche von Basophilen lokalisiertes Polypeptid bindet, an einem festen Träger immobilisiert ist,
wobei es sich bei der chemilumineszenzfähigen Markierung um ein Enzym handeln kann, das eine Chemilumineszenz-Reaktion katalysiert, oder um ein Molekül, das selbst unter geeigneten Bedingungen ein Chemilumineszenz-Signal erzeugt, wobei das Enzym im ersten Falle in Kombination mit einer chemilumineszenzfähigen Substratlösung im Kit enthalten ist,
und wobei der an der Oberfläche von Basophilen lokalisierte Marker charakteristisch für aktivierte Basophile ist,
optional ein Mittel zum Stoppen der Aktivierung der Basophilen,
optional ein Mittel zum Lysieren und/oder Entfernen von Erythrozyten.

17. Verwendung eines Liganden, der spezifisch gegen ein an der Zelloberfläche von Basophilen lokalisiertes Polypeptid bindet, zusammen mit einem Liganden, der gegen einen an der Oberfläche von Basophilen lokalisierten Marker bindet,
zur Diagnose einer Allergie oder zur Herstellung eines Kits zur Diagnose einer Allergie oder zum Screening von Kandidatenwirkstoffen für die Therapie einer Allergie gegen ein Allergen,
wobei der Ligand gegen den an der Oberfläche von Basophilen lokalisierten Marker zur Detektion eine chemilumineszenzfähige Markierung aufweist,
wobei es sich bei der chemilumineszenzfähigen Markierung um ein Enzym handeln kann, das eine Chemilumineszenz-Reaktion katalysiert, oder um ein Molekül, das selbst unter geeigneten Bedingungen ein Chemilumineszenz-Signal erzeugt, wobei das Enzym im ersten Falle in Kombination mit einer chemilumineszenzfähigen Substratlösung verwandt wird,
wobei der Ligand, der spezifisch gegen ein an der Zelloberfläche von Basophilen lokalisiertes Polypeptid bindet, an einem festen Träger immobilisiert ist,
und wobei der an der Oberfläche von Basophilen lokalisierte Marker charakteristisch für aktivierte Basophile ist.

## Claims

1. A method of diagnosing an allergy, comprising the steps of:
a) contacting basophils from a whole blood sample from a patient with an allergen in an aqueous solution in the presence of the whole blood, with a volume content of whole blood in the aqueous solution of at least 20 percent by volume, under conditions which allow activation of the basophils by the allergen,
b) enriching the basophils from step a) and
c) detecting any activated basophils,
wherein the detecting in step c) is achieved in that a marker for basophils located on the surface of the basophils is detected by chemiluminescence,
wherein in step b) basophils are enriched in that they bind to a ligand, which binds specifically to a polypeptide that is a marker characteristic of activated basophils,
and/or the detecting in step c) is achieved in that a marker characteristic of activated basophils is detected by chemiluminescence.

2. The method of claim 1, wherein the whole blood is unprocessed whole blood.

3. The method of any one of claims 1 to 2, wherein step a) is carried out in an aqueous solution with a whole blood content of at least 30, preferably 35, more preferably 40, more preferably 45 percent by volume.

4. The method of any one of claims 1 to 3, comprising the step of stopping the activation of the basophils, preferably before step b).

5. The method of any one of claims 1 to 4, wherein in step b), the basophils are enriched in that they bind to a ligand that binds specifically to a polypeptide located on the cell surface of basophils, preferably independently of their activation state.

6. The method of claim 5, wherein the ligand is a monoclonal antibody against the polypeptide.

7. The method of any of claims 1 to 6, wherein in step b), the basophils are immobilized.

8. The method of claim 7, wherein the basophils are immobilized on a bead, preferably a magnetic bead.

9. The method of any one of claims 7 to 8, wherein the basophils are washed after immobilization.

10. The method of any one of claims 1 to 9, wherein in step b), erythrocytes are lysed and removed.

11. The method of claim 10, wherein the erythrocytes are removed by centrifugation before immobilization of the basophils.

12. The method of any one of claims 1 to 11, wherein the detecting in step c) is achieved by using a ligand against the marker located on the surface of basophils provided with a label capable of chemiluminescence, preferably a marker that is characteristic of activated basophils.

13. The method of claim 12, wherein the ligand provided with a label capable of chemiluminescence is a monoclonal antibody that itself comprises a label capable of chemiluminescence or binds to a secondary antibody with a label capable of chemiluminescence.

14. The method of any one of claims 1 to 13, wherein steps a), b) and c) are carried out in a well of a microtiter plate.

15. The method of any one of claims 1 to 14, wherein step a) is carried out before steps b) and c).

16. A kit comprising
a vessel for bringing the basophils into contact with the allergen, wherein the vessel is preferably a microtiter plate,
optionally an allergen, and
a ligand binding specifically against a polypeptide located on the cell surface of basophils for the enrichment of basophils and a ligand against a marker located on the surface of basophils for detection,
wherein the ligand binding specifically against the marker located on the surface of basophils comprises a label capable of chemiluminescence for detection,
wherein the ligand binding specifically against a polypeptide located on the cell surface of basophils is immobilized on a solid support,
wherein the label capable of chemiluminescence is optionally an enzyme that catalyzes a chemiluminescence reaction or a molecule that itself produces a chemiluminescence signal under suitable conditions, wherein the enzyme in the former case is contained in the kit in combination with a substrate solution capable of chemiluminescence,
and wherein the marker located on the surface of basophils is characteristic of activated basophils,
optionally an agent for stopping activation of the basophils, and
optionally an agent for lysing and/or removing erythrocytes.

17. Use of a ligand that binds specifically against a polypeptide located on the cell surface of basophils, together with a ligand that binds against a marker located on the surface of basophils,
for the diagnosis of an allergy or for the preparation of a kit for the diagnosis of an allergy or for the screening of candidate substances for the therapy of an allergy to an allergen,
wherein the ligand against the marker located on the surface of basophils comprises a label capable of chemiluminescence for detection,
wherein the label capable of chemiluminescence is optionally an enzyme that catalyzes a chemiluminescence reaction or a molecule that itself produces a chemiluminescence signal under suitable conditions, wherein the enzyme in the former case is used in combination with a substrate solution capable of chemiluminescence,
wherein the ligand, which binds specifically against a polypeptide located on the cell surface of basophils, is immobilized on a solid support,
and wherein the marker located on the surface of basophils is characteristic of activated basophils.

## Revendications

1. Procédé de diagnostic d'une allergie, comprenant les étapes :
a) mise en contact de basophiles avec un échantillon de sang total d'un patient avec un allergène en solution aqueuse en présence du sang total, la teneur en volume de la solution aqueuse en le sang total étant d'au moins 20 pour-cent en volume, et dans des conditions qui autorisent une activation des basophiles par l'allergène,
b) enrichissement des basophiles de a) et
c) le décèlement des basophiles activés,
dans lequel le décèlement de l'étape c) est réalisé par le fait qu'un marqueur des basophiles, localisé sur la surface des basophiles, est détecté par chimioluminescence,
dans lequel, dans l'étape b), les basophiles sont enrichis par le fait qu'ils se lient à un ligand, qui se lie spécifiquement à un polypeptide qui est un marqueur caractéristique des basophiles activés,
et/ou le décèlement de l'étape c) est réalisé par le fait qu'un marqueur caractéristique des basophiles activés est détecté par chimioluminescence.

2. Procédé selon la revendication 1, dans lequel, pour ce qui concerne le sang total, il s'agit d'un sang total non traité.

3. Procédé selon l'une des revendications 1 à 2, dans lequel l'étape a) est mise en œuvre en solution aqueuse, la teneur en volume en le sang total étant d'au moins 30, de préférence de 35, plus préférentiellement de 40, plus préférentiellement de 45 pour-cent en volume.

4. Procédé selon l'une des revendications 1 à 3, comprenant les étapes d'interruption de l'activation des basophiles, de préférence avant l'étape b).

5. Procédé selon l'une des revendications 1 à 4, dans lequel, dans l'étape b), les basophiles sont enrichis par le fait qu'ils se lient à un ligand qui se lie spécifiquement à un polypeptide qui est, dans le cas des basophiles, localisé sur la surface cellulaire, de préférence indépendamment de leur état d'activation.

6. Procédé selon la revendication 5, dans lequel le ligand est un anticorps monoclonal contre le polypeptide.

7. Procédé selon l'une des revendications 1 à 6, dans lequel, dans l'étape b), les basophiles subissent une immobilisation.

8. Procédé selon la revendication 7, dans lequel les basophiles sont immobilisés sur une perle, de préférence une perle magnétique.

9. Procédé selon l'une des revendications 7 à 8, dans lequel les basophiles sont lavés après l'immobilisation.

10. Procédé selon l'une des revendications 1 à 9, dans lequel, dans l'étape b), des érythrocytes sont lysés et éliminés.

11. Procédé selon la revendication 10, dans lequel les érythrocytes sont éliminés par centrifugation avant l'immobilisation des basophiles.

12. Procédé selon les revendications 1 à 11, dans lequel le décèlement de l'étape c) est mis en œuvre à l'aide d'un ligand, pourvu d'un marquage chimioluminescent, contre le marqueur localisé sur la surface des basophiles, de préférence un marqueur qui est caractéristique des basophiles activés.

13. Procédé selon la revendication 12, dans lequel, pour ce qui concerne les ligands pourvus d'un marquage chimioluminescent, il s'agit d'un anticorps monoclonal qui luimême présente un marquage chimioluminescent, ou auquel se lie un anticorps secondaire comportant un marquage chimioluminescent.

14. Procédé selon les revendications 1 à 13, dans lequel les étapes a), b) et c) sont mises en œuvre dans un puits d'une plaque de microtitrage.

15. Procédé selon les revendications 1 à 14, dans lequel l'étape a) est mise en œuvre avant les étapes b) et c).

16. Kit comprenant
un récipient pour la mise en contact des basophiles avec l'allergène, le récipient étant de préférence une plaque de microtitrage,
éventuellement un allergène,
un ligand qui se lie spécifiquement à un polypeptide localisé sur la surface cellulaire de basophiles, pour l'enrichissement de basophiles, et un ligand d'un marqueur localisé sur la surface de basophiles, pour la détection,
le ligand qui se lie spécifiquement au marqueur localisé sur la surface de basophiles présentant pour la détection un marquage chimioluminescent,
le ligand qui se lie spécifiquement à un polypeptide localisé sur la surface cellulaire de basophiles étant immobilisé sur un support solide,
pour ce qui concerne le marquage chimioluminescent, il peut s'agir d'une enzyme qui catalyse une réaction de chimioluminescence, ou d'une molécule qui elle-même, dans des conditions appropriées, produit un signal de luminescence, l'enzyme, dans le premier cas, étant contenue dans le kit en combinaison avec une solution de substrat chimioluminescente,
et le marqueur localisé sur la surface de basophiles étant caractéristique des basophiles activés,
éventuellement un moyen pour interrompre l'activation des basophiles,
éventuellement un moyen pour lyser et/ou éliminer les érythrocytes.

17. Utilisation d'un ligand qui se lie spécifiquement à un polypeptide localisé sur la surface cellulaire de basophiles, conjointement à un ligand qui se lie à un marqueur localisé sur la surface de basophiles,
pour le diagnostic d'une allergie ou pour la fabrication d'un kit destiné au diagnostic d'une allergie ou pour le criblage de principes actifs candidats pour la thérapie d'une allergie à un allergène,
le ligand du marqueur localisé sur la surface de basophiles présentant pour la détection un marquage chimioluminescent,
pour ce qui concerne le marquage chimioluminescent, il peut s'agir d'une enzyme qui catalyse la réaction de chimioluminescence, ou d'une molécule qui elle-même dans des conditions appropriées, produit un signal de luminescence, l'enzyme, dans le premier cas, étant utilisée en combinaison avec une solution de substrat chimioluminescente,
le ligand qui se lie spécifiquement à un polypeptide localisé sur la surface cellulaire de basophiles étant immobilisé sur un support solide,
et le marqueur localisé sur la surface de basophiles étant caractéristique des basophiles activés.
